# EUROPEAN PATENT APPLICATION

(11) **EP 4 095 157 A1**
(43) Date of publication of application: **30.11.2022**
(21) Application number: 21743733.4
(22) Date of filing: 22.01.2021
(51) Int. Cl.: C07K 16/22, A61K 39/395

(54) **ANTI-ANGPTL3 ANTIBODY AND USE THEREOF**

(30) Priority: 22.01.2020 CN 202010073192
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: FU, Yayuan, Shanghai 200245 (CN); XU, Yingxia, Shanghai 200245 (CN); LIN, Bing, Shanghai 200245 (CN); TAO, Weikang, Shanghai 200245 (CN)
(74) Representative: Sonzogni, Laura Gabriella
(86) International application number: PCT/CN2021/073239
(87) International publication number: WO 2021/147984

(57) **Abstract**

The present invention relates to an anti-ANGPTL3 antibody and a use thereof. In particular, the present invention relates to the anti-ANGPTL3 antibody and an antigen-binding fragment thereof, or a pharmaceutically acceptable salt or solvent thereof, and a use thereof as a medicine, in particular a use in the preparation of a medicine for treating hyperlipidemia.

## Description

### TECHNICAL FIELD

The present disclosure relates to the field of antibody drugs. Specifically, an anti-ANGPTL3 antibody drug and use thereof are included.

### BACKGROUND

The statement herein merely provide background information related to the present invention and may not necessarily constitute the prior art.

The angiopoietin-like protein 3 (ANGPTL3, ANGPTL-3) gene is a novel gene identified by Conklin et al. from EST databases based on signal sequences and amphipathic helices, and they isolated the full-length cDNA of human ANGPTL3 from a human fetal liver/spleen cDNA library (Conklin et al., 1999, Genomics 62: 477-482). The 460-amino acid human ANGPTL3 protein (hANGPTL3) shares 76% amino acid sequence identity with mouse ANGPTL3 protein and has the characteristic structure of angiopoietins: a signal peptide, an extended helical domain predicted to form dimeric or trimeric coiled-coils, a short linker peptide, and a globular fibrinogen homology domain (FD) (Conklin et al., 1999, Genomics 62: 477-482). ANGPTL3 is different from angiopoietins (ANGs), and ANGPTL3 does not bind to Tie2. However, it can also induce angiogenesis by binding to integrin αvβ3 via its C-terminal FD (Camenisch et al., 2002, J Biol Chem 277: 17281-17290).

In addition, ANGPTL3 is an important factor affecting fat metabolism. ANGPTL3 can inhibit LPL activity, thereby reducing clearance of triglycerides and low density lipoproteins, while hypertriglyceridemia is involved in the pathogenesis of a range of metabolic diseases. Elevated plasma triglyceride level can be implicated in the pathological process of atherosclerosis by inducing platelet aggregation and the production of plasminogen activator inhibitor-1 (PAI-1), increasing the number of foam cells and promoting the proliferation and migration of vascular smooth muscle cells. The anti-ANGPTL3 antibody can specifically bind to ANGPTL3, so that the ANGPTL3 activity can be inhibited or interfered with, and thus diseases such as atherosclerosis and hyperlipidemia can be treated. At present, various anti-ANGPTL3 antibodies have been disclosed in patent documents such as WO2012174178A1, WO2008073300A2 and CN107085112A.

### SUMMARY

The present disclosure provides an anti-ANGPTL3 antibody or an antigen-binding fragment thereof. In some embodiments, the present disclosure provides an anti-ANGPTL3 antibody or an antigen-binding fragment thereof, which comprises a heavy chain variable region and a light chain variable region of an antibody, wherein:
i) the heavy chain variable region comprises an HCDR1, an HCDR2 and an HCDR3 identical in sequences to those of a heavy chain variable region set forth in SEQ ID NO: 9, and the light chain variable region comprises an LCDR1, an LCDR2 and an LCDR3 identical in sequences to those of a light chain variable region set forth in SEQ ID NO: 10;
ii) the heavy chain variable region comprises an HCDR1, an HCDR2 and an HCDR3 identical in sequences to those of a heavy chain variable region set forth in SEQ ID NO: 11, and the light chain variable region comprises an LCDR1, an LCDR2 and an LCDR3 identical in sequences to those of a light chain variable region set forth in SEQ ID NO: 12; or
iii) the heavy chain variable region comprises an HCDR1, an HCDR2 and an HCDR3 identical in sequences to those of a heavy chain variable region set forth in SEQ ID NO: 13, and the light chain variable region comprises an LCDR1, an LCDR2 and an LCDR3 identical in sequences to those of a light chain variable region set forth in SEQ ID NO: 14.

In some embodiments, provided is the anti-ANGPTL3 antibody or the antigen-binding fragment thereof described above, which comprises a heavy chain variable region and a light chain variable region, wherein:
iv) the heavy chain variable region comprises an HCDR1, an HCDR2 and an HCDR3 having sequences set forth in SEQ ID NO: 18, SEQ ID NO: 19 and SEQ ID NO: 20, respectively, and the light chain variable region comprises an LCDR1, an LCDR2 and an LCDR3 having sequences set forth in SEQ ID NO: 15, SEQ ID NO: 16 and SEQ ID NO: 17, respectively;
v) the heavy chain variable region comprises an HCDR1, an HCDR2 and an HCDR3 having sequences set forth in SEQ ID NO: 24, SEQ ID NO: 25 and SEQ ID NO: 26, respectively, and the light chain variable region comprises an LCDR1, an LCDR2 and an LCDR3 having sequences set forth in SEQ ID NO: 21, SEQ ID NO: 22 and SEQ ID NO: 23, respectively; or
vi) the heavy chain variable region comprises an HCDR1, an HCDR2 and an HCDR3 having sequences set forth in SEQ ID NO: 24, SEQ ID NO: 28 and SEQ ID NO: 26, respectively, and the light chain variable region comprises an LCDR1, an LCDR2 and an LCDR3 having sequences set forth in SEQ ID NO: 27, SEQ ID NO: 22 and SEQ ID NO: 23, respectively. In some embodiments, provided is the anti-ANGPTL3 antibody or the antigen-binding fragment thereof described above, which comprises a heavy chain variable region and a light chain variable region, wherein:
   (A) the heavy chain variable region has at least 90%, 91%, 92%, 93%, 94%, 95%, 96%, 97%, 98% or 99% identity to a sequence set forth in SEQ ID NO: 9, and/or the light chain variable region has at least 90%, 91%, 92%, 93%, 94%, 95%, 96%, 97%, 98% or 99% identity to a sequence set forth in SEQ ID NO: 10;
   (B) the heavy chain variable region has at least 90%, 91%, 92%, 93%, 94%, 95%, 96%, 97%, 98% or 99% identity to a sequence set forth in SEQ ID NO: 11, and/or the light chain variable region has at least 90%, 91%, 92%, 93%, 94%, 95%, 96%, 97%, 98% or 99% identity to a sequence set forth in SEQ ID NO: 12; or
   (C) the heavy chain variable region has at least 90%, 91%, 92%, 93%, 94%, 95%, 96%, 97%, 98% or 99% identity to a sequence set forth in SEQ ID NO: 13, and/or the light chain variable region has at least 90%, 91%, 92%, 93%, 94%, 95%, 96%, 97%, 98% or 99% identity to a sequence set forth in SEQ ID NO: 14.

In some embodiments, provided is the anti-ANGPTL3 antibody or the antigen-binding fragment thereof described above, which comprises a heavy chain variable region and a light chain variable region shown below:
(D) the heavy chain variable region has a sequence set forth in SEQ ID NO: 9, and the light chain variable region has a sequence set forth in SEQ ID NO: 10;
(E) the heavy chain variable region has a sequence set forth in SEQ ID NO: 11, and the light chain variable region has a sequence set forth in SEQ ID NO: 12; or
(F) the heavy chain variable region has a sequence set forth in SEQ ID NO: 13, and the light chain variable region has a sequence set forth in SEQ ID NO: 14.

In some embodiments, provided is the anti-ANGPTL3 antibody or the antigen-binding fragment thereof described above, wherein the anti-ANGPTL3 antibody further comprises a heavy chain constant region and a light chain constant region; in some embodiments, the heavy chain constant region is selected from the group consisting of human IgG1, IgG2, IgG3 and IgG4 constant regions, and the light chain constant region is selected from the group consisting of human κ chain and λ chain constant regions; in some embodiments, the heavy chain constant region has a sequence set forth in SEQ ID NO: 29 or a sequence having at least 85%, 86%, 87%, 88%, 89%, 90%, 91%, 92%, 93%, 94%, 95%, 96%, 97%, 98% or 99% sequence identity to the sequence set forth in SEQ ID NO. 29, and/or the light chain constant region has a sequence set forth in SEQ ID NO: 30 or a sequence having at least 85%, 86%, 87%, 88%, 89%, 90%, 91%, 92%, 93%, 94%, 95%, 96%, 97%, 98% or 99% sequence identity to the sequence set forth in SEQ ID NO. 30. In some embodiments, provided is the anti-ANGPTL3 antibody or the antigen-binding fragment thereof described above, wherein the anti-ANGPTL3 antibody comprises a heavy chain and a light chain, wherein,
(C) the heavy chain has at least 85%, 86%, 87%, 88%, 89%, 90%, 91%, 92%, 93%, 94%, 95%, 96%, 97%, 98% or 99% identity to a sequence set forth in SEQ ID NO: 31, and/or the light chain has at least 85%, 86%, 87%, 88%, 89%, 90%, 91%, 92%, 93%, 94%, 95%, 96%, 97%, 98% or 99% identity to a sequence set forth in SEQ ID NO: 32; (H) the heavy chain has at least 85%, 86%, 87%, 88%, 89%, 90%, 91%, 92%, 93%, 94%, 95%, 96%, 97%, 98% or 99% identity to a sequence set forth in SEQ ID NO: 33, and/or the light chain has at least 85%, 86%, 87%, 88%, 89%, 90%, 91%, 92%, 93%, 94%, 95%, 96%, 97%, 98% or 99% identity to a sequence set forth in SEQ ID NO: 34; or
(I) the heavy chain has at least 85%, 86%, 87%, 88%, 89%, 90%, 91%, 92%, 93%, 94%, 95%, 96%, 97%, 98% or 99% identity to a sequence set forth in SEQ ID NO: 35, and/or the light chain has at least 85%, 86%, 87%, 88%, 89%, 90%, 91%, 92%, 93%, 94%, 95%, 96%, 97%, 98% or 99% identity to a sequence set forth in SEQ ID NO: 36.

In some embodiments, provided is the anti-ANGPTL3 antibody or the antigen-binding fragment thereof described above, wherein the anti-ANGPTL3 antibody comprises a heavy chain and a light chain, wherein,
(J) the heavy chain has a sequence set forth in SEQ ID NO: 31, and the light chain has a sequence set forth in SEQ ID NO: 32;
(K) the heavy chain has a sequence set forth in SEQ ID NO: 33, and the light chain has a sequence set forth in SEQ ID NO: 34; or
(L) the heavy chain has a sequence set forth in SEQ ID NO: 35, and the light chain has a sequence set forth in SEQ ID NO: 36.

In some embodiments, the present disclosure provides an isolated anti-ANGPTL3 antibody or an antigen-binding fragment thereof, wherein the anti-ANGPTL3 antibody or the antigen-binding fragment thereof competes with an anti-ANGPTL3 antibody or the antigen-binding fragment thereof described above for binding to an ANGPTL3 antigen. In some embodiments, the ANGPTL3 antigen is human ANGPTL3, mouse ANGPTL3, monkey ANGPTL3 or rat ANGPTL3.

In some embodiments, an antibody described herein is a human antibody or a human-derived antibody. In some embodiments, the antigen-binding fragment described herein is selected from the group consisting of Fab, F(ab')2, Fab', Fd, Fv, dsFv, scFv, Fab and a diabody. In some embodiments, the anti-ANGPTL3 antibody or the antigen-binding fragment thereof of the present disclosure has at least one of the following characteristics a-d:
a. Specifically binding to an ANGPTL3 protein. In some embodiments, the anti-ANGPTL3 antibody or the antigen-binding fragment thereof binds to human ANGPTL3, mouse ANGPTL3, cynomolgus monkey ANGPTL3 and/or rat ANGPT3L with an EC₅₀ of less than 10 nM (e.g., less than or equal to 5 nM, less than or equal to 3 nM, less than or equal to 1 nM, less than or equal to 0.8 nM, less than or equal to 0.6 nM, less than or equal to 0.4 nM, less than or equal to 0.3 nM, or less than or equal to 0.2 nM); in some embodiments, the EC₅₀ is determined by the ELISA method, e.g., as described in Test Example 1 of the present disclosure; in some embodiments, the EC₅₀ is determined by the HTRF method, e.g., as described in Test Example 2 of the present disclosure;
b. Binding to an ANGPTL3 protein with high affinity. In some embodiments, the anti-ANGPTL3 antibody or the antigen-binding fragment thereof binds to human ANGPTL3, mouse ANGPTL3, cynomolgus monkey ANGPTL3 and/or rat ANGPTL3 antigen with a KD value of less than 10E-10 M (e.g., less than or equal to 9E-10 M, less than or equal to 8E-10 M, less than or equal to 7E-10 M, less than or equal to 6E-10 M, less than or equal to 3E-10 M, less than or equal to 2E-10 M, less than or equal to 1E-10 M, less than or equal to 8E-11 M, less than or equal to 6E-11 M, or less than or equal to 5E-11 M); in some embodiments, the KD value is determined by the Biacore method, e.g., as described in Test Example 4 of the present disclosure;
c. Blocking the inhibition of ANGPTL3 against LPL enzyme. In some embodiments, the anti-ANGPTL3 antibody or the antigen-binding fragment thereof blocks the inhibition of human ANGPTL3, mouse ANGPTL3, cynomolgus monkey ANGPTL3 and/or rat ANGPTL3 against LPL enzyme activity with an IC₅₀ value of less than 170 nM (e.g., less than or equal to 80 nM, less than or equal to 135 nM, less than or equal to 80 nM, or less than or equal to 55 nM). The IC₅₀ can be determined by the enzyme activity assay, e.g., as described in Test Example 3 of the present disclosure; and/or
d. Exhibiting good lipid lowering effect. In some embodiments, the lipid lowering effect of the anti-ANGPTL3 antibody or the antigen-binding fragment thereof of the present disclosure is shown in Test Examples 5, 6 and/or 7. In some embodiments, the present disclosure provides a nucleic acid molecule encoding the anti-ANGPTL3 antibody or the antigen-binding fragment thereof described above.

In some embodiments, the present disclosure provides a vector comprising the nucleic acid molecule described above.

In some embodiments, the present disclosure provides a host cell comprising the nucleic acid molecule described above. In some embodiments, the present disclosure provides a host cell obtained by transformation (or transduction or transfection) with the vector described above; the host cell is selected from the group consisting of prokaryotic cells and eukaryotic cells, preferably eukaryotic cells, more preferably mammalian cells. In some embodiments, the host cell does not include any human cells capable of developing into a whole individual, such as human embryonic stem cells, fertilized eggs or germ cells; in some embodiments, the host cell is a eukaryotic cell, more preferably a mammalian cell including but not limited to CHO, 293, NSO, and a mammalian cell in which gene editing (e.g., knocking out genes such as FUT8 or GnT-III) can change the glycosylation modification of the antibody or the antigen-binding fragment thereof and thereby change the ADCC function of the antibody or the antigen-binding fragment thereof; in some embodiments, the mammalian cell does not include a human cell.

In some embodiments, the present disclosure provides a pharmaceutical composition comprising a therapeutically effective amount of the anti-ANGPTL3 antibody or the antigen-binding fragment thereof described above or the nucleic acid molecule described above, and one or more pharmaceutically acceptable carriers, diluents, buffers or excipients. In some embodiments, the pharmaceutical composition comprises, per unit dose, 0.01 wt. % to 99 wt. % of the anti-ANGPTL3 antibody or the antigen-binding fragment thereof described above or the nucleic acid molecule described above. In some embodiments, the pharmaceutical composition comprises, per unit dose, 0.1-2000 mg, more preferably 1-1000 mg, of the anti-ANGPTL3 antibody or the antigen-binding fragment thereof described above or the nucleic acid molecule described above.

In some embodiments, the present disclosure provides a method for immunodetection or determination of ANGPTL3, which comprises the anti-ANGPTL3 antibody or the antigen-binding fragment thereof described above. In another aspect, the present disclosure provides a method for detecting the expression level of ANGPTL3 in a sample from a subject *in vitro,* and the method comprises the step of contacting the anti-ANGPTL3 antibody or the antigen-binding fragment thereof described in the present disclosure with the sample; in some embodiments, the sample is a plasma, serum or whole blood sample from the subject. In some embodiments, the expression level of ANGPTL3 can be detected using any method known to those skilled in the art, including but not limited to Western blot, immunoblot, ELISA and mass spectrometry. In some embodiments, the present disclosure provides a kit comprising the anti-ANGPTL3 antibody or the antigen-binding fragment thereof described above.

In some embodiments, the present disclosure provides a method for preparing the anti-ANGPTL3 antibody or the antigen-binding fragment thereof described above.

In some embodiments, the present disclosure provides use of the anti-ANGPTL3 antibody or the antigen-binding fragment thereof described above in preparing a reagent for immunodetection of ANGPTL3.

In some embodiments, the present disclosure provides the anti-ANGPTL3 antibody or the antigen-binding fragment thereof described above for use in immunodetection or determination of ANGPTL3.

In some embodiments, the present disclosure provides a kit comprising the anti-ANGPTL3 antibody or the antigen-binding fragment thereof described above. In some embodiments, the present disclosure provides a kit for detecting the expression level of ANGPTL3, which comprises the anti-ANGPTL3 antibody or the antigen-binding fragment thereof described above. In some embodiments, the kit is used in the method for detecting the expression level of ANGPTL3 described herein; in some embodiments, the detection method is any method known to those skilled in the art, including but not limited to Western blot, immunoblot, ELISA and mass spectrometry. In some embodiments, the present disclosure provides a method for treating a disease or a disorder, which comprises administering to a subject a therapeutically effective amount of the anti-ANGPTL3 antibody or the antigen-binding fragment thereof, the nucleic acid molecule or the pharmaceutical composition described above; in some embodiments, the disease or the disorder is an ANGPTL3-associated disease or disorder; in some embodiments, the disease or the disorder is hypercholesterolemia, hyperlipidemia or an atherosclerotic disease.

In some embodiments, the present disclosure provides use of the anti-ANGPTL3 antibody or the antigen-binding fragment thereof, the nucleic acid molecule or the pharmaceutical composition described above in preparing a medicament for treating a disease or a disorder. In some embodiments, the disease or the disorder is an ANGPTL3-associated disease or disorder; in some embodiments, the disease or the disorder is hypercholesterolemia, hyperlipidemia or an atherosclerotic disease.

In some embodiments, the present disclosure provides the anti-ANGPTL3 antibody or the antigen-binding fragment thereof, the nucleic acid molecule or the pharmaceutical composition described above for use as a medicament. In some embodiments, the medicament is used for treating an ANGPTL3-associated disease or disorder; in some embodiments, the disease or the disorder is hypercholesterolemia, hyperlipidemia or an atherosclerotic disease.

### Detailed Description of the Invention

### Terms (definition)

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. The three-letter and single-letter codes for amino acids used in the present disclosure are as described in J. biol. chem, 243, p3558 (1968).

The "antibody" described herein refers to an immunoglobulin, and a natural intact antibody is of a tetrapeptide chain structure formed by connection between two identical heavy chains and two identical light chains by interchain disulfide bonds. According to differences in the amino acid composition and the order of arrangement of the heavy chain constant regions, immunoglobulins can be divided into five classes, otherwise called isotypes of immunoglobulins, namely IgM, IgD, IgG, IgA and IgE, with their corresponding heavy chains being µ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG may be divided into IgG1, IgG2, IgG3 and IgG4. Light chains are classified into κ or λ chains by the differences in the constant regions. Each of the five classes of Ig may have a κ chain or λ chain.

In the heavy and light chains of antibodies, the sequences of about 110 amino acids near the N-terminal vary considerably and thus are referred to as variable regions (Fv regions); the remaining amino acid sequences near the C-terminal are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (VL) or heavy chain variable region (VH) consists of 3 CDRs and 4 FRs arranged from the amino-terminal to the carboxy-terminal in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3.

Besides full-length antibodies, the "antibodies" described herein also include antigen-binding fragments capable of binding to an antigen.

The antibodies described herein include human antibodies.

The "murine antibody" described herein is a monoclonal antibody against human ANGPTL3 prepared according to the knowledge and skill in the art. At the time of preparation, ANGPTL3 antigen is injected into test subjects, and then hybridomas expressing an antibody with the desired sequence or functional properties are isolated. In one preferred embodiment of the present disclosure, the murine anti-ANGPTL3 antibody or the antigen-binding fragment thereof may further comprise a light chain constant region of a murine κ or λ chain or a variant thereof, or further comprise a heavy chain constant region of a murine IgG1, IgG2, or IgG3 or a variant thereof.

The term "chimeric antibody" described herein refers to an antibody obtained by fusing a variable region of a heterologous (e.g., murine) antibody and a constant region of a parent antibody (e.g., human antibody), which can alleviate an immune response induced by the heterologous antibody. For example, the human-murine chimeric antibody is established by firstly establishing hybridoma secreting murine specific monoclonal antibody, then cloning a variable region gene from the mouse hybridoma cells, cloning a constant region gene of human antibody as required, connecting the mouse variable region gene and the human constant region gene into a chimeric gene, inserting the chimeric gene into an expression vector, and finally expressing chimeric antibody molecules in a eukaryotic system or prokaryotic system. In a preferred embodiment of the present disclosure, the antibody light chain of the anti-ANGPTL3 chimeric antibody further comprises a light chain constant region of a human κ or λ chain or a variant thereof. The antibody heavy chain of the ANGPTL3 chimeric antibody further comprises a heavy chain constant region of human IgG1, IgG2, IgG3 or IgG4 or a variant thereof, preferably a heavy chain constant region of human IgG1, IgG2 or IgG4, or an IgG1, IgG2 or IgG4 variant using an amino acid mutation (e.g., an L234A and/or L235A mutation, and/or an S228P mutation).

The term "humanized antibody", also referred to as CDR-grafted antibody, refers to an antibody produced by grafting mouse CDR sequences into a human antibody variable region framework, i.e., an antibody produced in a different type of human germline antibody framework sequence. Therefore, the heterogeneous reaction induced by the presence of a large number of mouse protein components in the chimeric antibody can be overcome. Such framework sequences can be obtained from public DNA databases or disclosed references that include germline antibody gene sequences. For example, germline DNA sequences of genes of the human heavy and light chain variable regions can be found in the "VBase" human germline sequence database, as well as in Kabat, E. A. et al., 1991 Sequences of Proteins of Immunological Interest, 5th edition. To avoid decreased activity caused by decreased immunogenicity, the variable region framework sequence of the human antibody may be subjected to a minimum reverse mutation or back mutation to retain activity. The humanized antibody of the present disclosure also include humanized antibodies which were further subjected to CDR affinity maturation mutation by yeast display.

The terms "human antibody" and "human-derived antibody" are used interchangeably and mean that one or more of the variable and constant regions are derived from human immunoglobulin sequences. One preferred way is that all of the variable and constant regions are derived from human immunoglobulin sequences, i.e., "fully human-derived antibodies" or "fully human antibodies". These antibodies can be obtained in a variety of ways, including antibodies obtained by isolating B cells from human PBMC, spleen and lymph node tissue and constructing natural single-stranded phage human antibody library by using phage display technology, or by immunizing transgenic mice capable of expressing human antibody light and heavy chains and screening. The human antibodies of the present disclosure also include antibodies that still bind to the antigen of interest and are obtained by mutation of one or more amino acids on the basis of human antibodies.

The "conventional variant" of the human antibody heavy chain constant region and the human antibody light chain constant region described herein refers to the variant of heavy chain constant region or light chain constant region derived from human that has been disclosed in the prior art and does not change the structure and function of the antibody variable region. Exemplary variants include IgG1, IgG2, IgG3 or IgG4 heavy chain constant region variants with site-directed modifications and amino acid substitutions in the heavy chain constant region. Specific substitutions are, for example, YTE mutation, L234A and/or L235A mutation, or S228P mutation, or mutations to obtain a knob-into-hole structure (so that the antibody heavy chain has a combination of knob-Fc and hole-Fc) known in the art. These mutations have been confirmed to make the antibody have new properties, but do not change the function of the antibody variable region.

The terms "full-length antibody," "intact antibody," "complete antibody," and "whole antibody" are used interchangeably herein to refer to an antibody in its substantially intact form, as distinguished from an antigen-binding fragment defined below. The term especially refers to antibodies in which the light and heavy chains comprise constant regions. The "antibody" of the present disclosure include "full-length antibodies" and antigen-binding fragments thereof.

In some embodiments, the full-length antibodies of the present disclosure include full-length antibodies formed by linking a light chain variable region to a light chain constant region and linking a heavy chain variable region to a heavy chain constant region. Those skilled in the art can select different antibody-derived light chain constant regions and heavy chain constant regions according to actual needs, for example, human antibody-derived light chain constant regions and heavy chain constant regions.

The term "antigen-binding fragment" or "functional fragment" refers to one or more fragments of the antibody that retain the ability to specifically bind to an antigen (e.g., ANGPTL3). It has been shown that a fragment of a full-length antibody can be used to perform the antigen-binding function of the antibody. Examples of the binding fragment included in the term "antigen-binding fragment" include (i) Fab fragment, a monovalent fragment consisting of VL, VH, CL and CH1 domains; (ii) F(ab')₂ fragment, a bivalent fragment comprising two Fab fragments linked by a disulfide bridge in the hinge region; (iii) Fd fragment, consisting of VH and CH1 domains; (iv) Fv fragment, consisting of VH and VL domains of one arm of the antibody; (V) dsFv, a stable antigen-binding fragment formed by VH and VL via interchain disulfide bonds therebetween; (vi) diabody, bispecific antibody and multi-specific antibody, comprising such fragments as scFv, dsFv and Fab. Furthermore, although the two domains of the Fv fragment, VL and VH, are encoded by separate genes, they can be linked by a synthetic linker by recombination, so that it is capable of producing a single protein chain in which the VL and VH regions pair to form a monovalent molecule (referred to as single-chain Fv (scFv); see, e.g., Bird et al. (1988) Science 242:423-426; and Huston et al. (1988) Proc. Natl. Acad. Sci USA 85:5879-5883). Such single-chain antibodies are also included in the term "antigen-binding fragment" of an antibody. Such antibody fragments are obtained using conventional techniques known to those skilled in the art, and screened for utility in the same manner as for intact antibodies. Antigen-binding portions may be produced using recombinant DNA technology or by enzymatic or chemical cleavage of intact immunoglobulins. Antibodies may be of different isotypes, e.g., IgG (e.g., subtype IgG1, IgG2, IgG3 or IgG4), IgA1, IgA2, IgD, IgE or IgM antibody.

Fab is an antibody fragment that has a molecular weight of about 50,000, has antigen-binding activity, and can be obtained by treating IgG antibody molecules with a protease papain (which cleaves the amino acid residue at position 224 of the H chain), in which about half of the H chain of the N-terminal side and the entire L chain are joined together by disulfide bonds.

F(ab')₂ is an antibody fragment that has a molecular weight of about 100,000, has antigen-binding activity, comprises two Fab regions connected at the hinge position, and can be obtained by digesting the lower part of the two disulfide bonds in the hinge region of IgG with the enzyme pepsin.

Fab' is an antibody fragment that has a molecular weight of about 50,000, has antigen-binding activity, and can be obtained by cleaving the disulfide bond in the hinge region of the F(ab')₂ described above. The Fab' of the present disclosure can be produced by using reducing agents, for example dithiothreitol, to treat the F(ab')₂ of the present disclosure which specifically recognizes ANGPTL3 and binds to the amino acid sequence of the extracellular region or three-dimensional structure thereof.

In addition, the Fab' may be produced by inserting DNA encoding the Fab' fragment of an antibody into a prokaryotic or eukaryotic expression vector and introducing the vector into a prokaryote or a eukaryote to express the Fab'.

The term "single-chain antibody", "single-chain Fv" or "scFv" refers to a molecule comprising an antibody heavy chain variable domain (or region; VH) and an antibody light chain variable domain (or region; VL) linked by a linker. Such scFv molecules may have a general structure: NH₂-VL-linker-VH-COOH or NH₂-VH-linker-VL-COOH. Suitable linkers in the prior art consist of repeated GGGGS amino acid sequences or variants thereof, for example, 1-4 repeated variants (Holliger et al. (1993), Proc. Natl. Acad. Sci. USA 90:6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061, Kipriyanov et al. (1999), J. Mol. Biol. 293:41-56; and Roovers et al. (2001), Cancer Immunol.

A diabody is an antibody fragment in which scFv or Fab is dimerized, and is an antibody fragment with bivalent antigen-binding activity. In the bivalent antigen-binding activity, the two antigens can be identical or different.

Bispecific antibodies and multi-specific antibodies refer to an antibodies that can simultaneously bind to two or more antigens or antigenic determinants and comprise scFv or Fab fragments that can bind to ANGPTL3.

The diabody of the present disclosure can be produced by the following steps: obtaining the encoding cDNA of VH and VL of the monoclonal antibody of the present disclosure which specifically recognizes human ANGPTL3 and binds to the amino acid sequence of the extracellular region or a three-dimensional structure thereof, constructing the DNA encoding scFv so that the amino acid sequence length of the peptide linker is 8 residues or less, inserting the DNA into a prokaryotic expression vector or a eukaryotic expression vector, and then introducing the expression vector into a prokaryote or a eukaryote to express the diabody.

dsFv is obtained by linking polypeptides in which one amino acid residue in each VH and VL is substituted with a cysteine residue via disulfide bonds between the cysteine residues. The amino acid residues substituted with cysteine residues can be selected according to known methods (Protein Engineering, 7, 697 (1994)) based on prediction of the three-dimensional structure of the antibody.

The full-length antibody or the antigen-binding fragment of the present disclosure can be produced by the following steps: obtaining the encoding cDNA the antibody of the present disclosure which specifically recognizes human ANGPTL3 and binds to the amino acid sequence of the extracellular region or a three-dimensional structure thereof, constructing the DNA encoding the dsFv, inserting the DNA into a prokaryotic expression vector or a eukaryotic expression vector, and then introducing the expression vector into a prokaryote or a eukaryote to express the dsFv.

The term "amino acid difference" or "amino acid mutation" refers to the presence of amino acid changes or mutations in the variant protein or polypeptide compared with the original protein or polypeptide, including occurrence of 1, 2, 3 or more amino acid insertions, deletions or substitutions on the basis of the original protein or polypeptide. The term "antibody framework" or "FR" refers to a portion of a variable domain VL or VH, which serves as a framework for the antigen-binding loops (CDRs) of the variable domain. It is essentially a variable domain without CDRs.

The term "complementarity determining region," "CDR" or "hypervariable region" refers to one of the 6 hypervariable regions within the variable domain of an antibody which primarily contribute to antigen binding. In general, there are three CDRs (HCDR1, HCDR2 and HCDR3) in each heavy chain variable region and three CDRs (LCDR1, LCDR2 and LCDR3) in each light chain variable region. Any one of a variety of well-known schemes can be used to determine the amino acid sequence boundaries of the CDRs, including the "Kabat" numbering rules (see Kabat et al., (1991), "Sequences of Proteins of Immunological Interest", 5th edition, Public Health Service, National Institutes of Health, Bethesda, MD), "Chothia" numbering rules (see Al-Lazikani et al., (1997) JMB 273: 927-948) and ImMunoGenTics (IMGT) numbering rules (Lefranc M. P., Immunologist, 7, 132-136 (1999); Lefranc, M. P., et al., Dev. Comp. Immunol., 27, 55-77 (2003)), etc.

The term "epitope" or "antigenic determinant" refers to a site on an antigen (e.g., a specific site on an ANGPTL3 molecule) to which an immunoglobulin or an antibody specifically binds. Epitopes typically comprise at least 3, 4, 5, 6, 7, 8, 9, 10, 11, 12, 13, 14 or 15 contiguous or non-contiguous amino acids in a unique spatial conformation. See, e.g., Epitope Mapping Protocols in Methods in Molecular Biology, volume 66, G.E. Morris, Ed. (1996).

The terms "specific binding", "selective binding", "selectively bind to", "specifically bind to" and "capable of specifically binding to" refer to the binding of an antibody to an epitope on a predetermined antigen. In general, the antibody binds with an affinity (KD) of less than about 10⁻⁸ M, e.g., less than about 10⁻⁹ M, 10⁻¹⁰ M, or 10⁻¹¹ M, 10⁻¹² M or less.

The term "KD" refers to the dissociation equilibrium constant for specific antibody-antigen interaction. Generally, the antibody of the present disclosure binds to ANGPTL3 with a dissociation equilibrium constant (KD) of less than about 10⁻⁷ M, e.g., less than about 10⁻⁸ M or 10⁻⁹ M. For example, in the present disclosure, the affinity KD value of the antibody for the cell surface antigen is determined using the FACS method; in other embodiments, the affinity KD value of the antibody for the antigen is determined using the Biacore method; in other embodiments, binding of the antibody to the antigen is determined using the ELISA method.

The term "compete", when used in a case where antigen-binding proteins (e.g., neutralizing antigen-binding proteins or neutralizing antibodies) compete for the same epitope, refers to the competition between the antigen-binding proteins, which is determined by the following assays in which a test antigen-binding protein (e.g., an antibody or an immunologically functional fragment thereof) prevents or inhibits (e.g., reduces) specific binding of a reference antigen-binding protein (e.g., a ligand or a reference antibody) to a common antigen (e.g., ANGPTL3 antigen or a fragment thereof). Numerous types of competitive binding assays are available for determining whether an antigen-binding protein competes with another, such as: solid phase direct or indirect radioimmunoassay (RIA), solid phase direct or indirect enzyme immunoassay (EIA), and sandwich competition assay (see, e.g., Stahli et al., 1983, Methods in Enzymology 9:242-253); solid phase direct biotin-avidin EIA (see, e.g., Kirkland et al., 1986, J. Immunol. 137:3614-3619), solid phase direct labeled assay, and solid phase direct labeled sandwich assay (see, e.g., Harlow and Lane, 1988, Antibodies: A Laboratory Manual, Cold Spring Harbor Press); solid phase direct labeled RIA with 1-125 label (see, e.g., Morel et al., 1988, Molec. Immunol. 25:7-15); solid phase direct biotin-avidin EIA (see, e.g., Cheung, et al., 1990, Virology 176:546-552) and direct labeled RIA (Moldenhauer et al., 1990, Scand. J. Immunol. 32:77-82). In general, the assay relates to a use of a purified antigen binding to a solid surface or a cell bearing any of an unlabeled assayed antigen-binding protein and a labeled reference antigen-binding protein. Competitive inhibition is measured by measuring the amount of label bound to the solid surface or the cell in the presence of the assayed antigen-binding protein. In general, the assayed antigen-binding protein is present in an excessive amount. Antigen-binding proteins identified by the competitive assay (competitive antigen-binding proteins) include: an antigen-binding protein binding to the same epitope as a reference antigen-binding protein, and an antigen-binding protein binding to an adjacent epitope sufficiently close to a binding epitope of the reference antigen-binding protein, and the two epitopes spatially interfere with each other to prevent the binding. Other detailed information regarding the method for assaying competitive binding is provided in the examples herein. In general, when the competitive antigen-binding protein exists in an excessive amount, the specific binding of the reference antigen-binding protein to the common antigen will be inhibited (such as reduced) by at least 40-45%, 45-50%, 50-55%, 55-60%, 60-65%, 65-70%, 70-75% or 75% or more. In some cases, the binding is inhibited by at least 80-85%, 85-90%, 90-95%, 95-97% or 97% or more.

The term "nucleic acid molecule" as used herein refers to a DNA molecule and an RNA molecule. The nucleic acid molecule may be single-stranded or double-stranded, and is preferably a double-stranded DNA, a single-stranded mRNA or a modified mRNA. A nucleic acid is "operably linked" when it is placed into a functional relationship with another nucleic acid sequence. For example, a promoter or enhancer is operably linked to a coding sequence if it affects the transcription of the coding sequence.

The amino acid sequence "identity" refers to the percentage of amino acid residues shared by a first sequence and a second sequence, wherein in aligning the amino acid sequences, gaps are introduced, when necessary, to achieve maximum percent sequence identity, and any conservative substitution is not considered as part of the sequence identity. For the purpose of determining percent amino acid sequence identity, alignments can be achieved in a variety of ways that are well-known in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences. The term "expression vector" refers to a nucleic acid molecule capable of transporting another nucleic acid to which it has been linked. In one embodiment, the vector is a "plasmid" that refers to a circular double-stranded DNA loop into which additional DNA segments can be ligated. In another embodiment, the vector is a viral vector, wherein additional DNA segments may be ligated into the viral genome. The vectors disclosed herein are capable of autonomously replicating in a host cell into which they are introduced (e.g., bacterial vectors having a bacterial origin of replication and episomal mammalian vectors) or capable of integrating into the genome of a host cell after being introduced into the host cell and thus replicating with the host genome (e.g., non-episomal mammalian vectors).

Methods of producing and purifying antibodies and antigen-binding fragments are well known in the art, for example, those described in chapters 5-8 and 15 of Antibodies: A Laboratory Manual, Cold Spring Harbor Press. For example, mice can be immunized with human ANGPTL3 or a fragment thereof, and the obtained antibodies can be renatured and purified, and amino acid sequencing can be performed by using conventional methods. Antigen-binding fragments can likewise be prepared using conventional methods. The antibody or the antigen-binding fragment described in the present invention is genetically engineered to contain one or more additional human FRs in the non-human CDRs. Human FR germline sequences can be obtained at the website of ImMunoGeneTics (IMGT) by comparing the IMGT human antibody variable region germline gene database with the MOE software, or obtained from the immunoglobulin journal 2001ISBN012441351.

The term "host cell" refers to a cell into which an expression vector has been introduced. Host cells may include bacterial, microbial, plant or animal cells. Bacteria susceptible to transformation include members of the *Enterobacteriaceae* family, such as strains of *Escherichia coli* or *Salmonella*; members of the *Bacillaceae* family, such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell line), 293 cells and NS0 cells. The engineered antibody or antigen-binding fragment of the present disclosure can be prepared and purified using conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of the antibody, particularly at the highly conservative N-terminal site of the Fc region. Stable clones are obtained by expression of the antibody that specifically binds to human ANGPTL3. Positive clones are expanded in serum-free medium in a bioreactor to produce the antibody. The culture solution with the secreted antibody can be purified using conventional techniques. For example, purification is carried out on an A or G Sepharose FF column containing an adjusted buffer. Non-specifically bound fractions are washed away. The bound antibody is eluted using pH gradient method, and the antibody fragments are detected using SDS-PAGE and collected. The antibody can be filtered and concentrated using conventional methods. Soluble mixtures and polymers can also be removed using conventional methods, such as molecular sieves and ion exchange. The resulting product needs to be immediately frozen, e.g., at -70 °C, or lyophilized.

"Administrating" "giving" and "treating", when applied to animals, humans, experimental subjects, cells, tissues, organs or biological fluid, refer to contact of an exogenous drug, a therapeutic agent, a diagnostic agent or composition with the animals, humans, subjects, cells, tissues, organs or biological fluid. "Administrating" "giving" and "treating" can refer to, for example, therapeutic, pharmacokinetic, diagnostic, research and experimental methods. The treatment of the cells comprises contacting the reagent with the cells and contacting the reagent with fluid, wherein the fluid are in contact with the cells. "Administrating" "giving" and "treating" also refer to treating, e.g., cells by reagents, diagnosis, binding compositions or by another cell *in vitro* and *ex vivo.* "Treating", when applied to human, veterinary or research subjects, refers to therapeutic treatment, preventive or prophylactic measures, and research and diagnostic applications.

"Treating" or "treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Typically, the therapeutic agent is administered in an amount effective to alleviate one or more symptoms of the disease in the patient or population being treated to induce regression of such symptoms or inhibiting the development of such symptoms to any clinically measurable degree. The amount of therapeutic agent effective to alleviate the symptoms of any particular disease (also referred to as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example treatment methods or products) may not be effective in alleviating all the target disease symptoms in every patient, as determined according to any statistical testing methods known in the art, such as Student t-test, chi-square test, Mann and Whitney's U test, Kruskal-Wallis test (H test), Jonckheere-Terpstra test and Wilcoxon test, they should reduce the target disease symptoms in a statistically significant number of patients.

"Conservative modification" or "conservative replacement or substitution" refers to replacement of amino acids in a protein with other amino acids having similar characteristics (e.g., charge, side-chain size, hydrophobicity/hydrophilicity, or backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)). In addition, the replacement of amino acids with similar structure or function is unlikely to disrupt the biological activity. Exemplary conservative substitutions are stated in the Table "Exemplary amino acid conservative substitutions" below.

**Table 1. Exemplary amino acid conservative substitutions**

| Original residue | Conservative substitution |
|---|---|
| Ala(A) | Gly; Ser |
| Arg(R) | Lys; His |
| Asn(N) | Gln; His; Asp |
| Asp(D) | Glu; Asn |
| Cys(C) | Ser; Ala; Val |
| Gln(Q) | Asn; Glu |
| Glu(E) | Asp; Gln |
| Gly(G) | Ala |
| His(H) | Asn; Gln |
| Ile(I) | Leu; Val |
| Leu(L) | Ile; Val |
| Lys(K) | Arg; His |
| Met(M) | Leu; Ile; Tyr |
| Phe(F) | Tyr; Met; Leu |
| Pro(P) | Ala |
| Ser(S) | Thr |
| Thr(T) | Ser |
| Trp(W) | Tyr; Phe |
| Tyr(Y) | Trp; Phe |
| Val(V) | Ile; Leu |

"Effective amount" or "effective dosage" refers to the amount of a drug, a compound or a pharmaceutical composition necessary to obtain any one or more beneficial or desired therapeutic results. For preventive use, the beneficial or desired results include elimination or reduction of risk, reduction of severity or delay of the onset of a disorder, including the biochemistry, histology and/or behavioral symptoms of the disorder, complications thereof and intermediate pathological phenotypes that appear during the progression of the disorder. For therapeutic applications, the beneficial or desired results include clinical results, such as reducing the incidence of various disorders related to the target antigen of the present disclosure or alleviating one or more symptoms of the disorder, reducing the dosage of other agents required to treat the disorder, enhancing the therapeutic effect of another agent, and/or delaying the progression of disorders of the patient related to the target antigen of the present disclosure.

"Exogenous" refers to substances produced outside organisms, cells or human bodies according to circumstances. "Endogenous" refers to substances produced inside cells, organisms or human bodies according to circumstances.

As used herein, the expressions "cell," "cell line," and "cell culture" are used interchangeably, and all such designations include their progenies. Therefore, the words "transformant" and "transformed cell" include primary test cells and cultures derived therefrom, regardless of the number of transfers. It should also be understood that all progenies may not be precisely identical in DNA content due to deliberate or unintentional mutations. Mutant progeny with the same function or biological activity as screened in the original transformed cells is included. When referring to different designations, they will become clear through the context.

"Polymerase chain reaction" or "PCR" as used herein refers to a procedure or technique in which a trace amount of a specific moiety of nucleic acid, RNA and/or DNA is amplified as described in, for example, US Patent No. 4,683,195. Generally speaking, it is necessary to obtain sequence information from the end or outside of the target region, so that oligonucleotide primers can be designed; these primers are the identical or similar in terms of sequence to the corresponding strand of the template to be amplified. The 5'-terminal nucleotide of 2 primers may coincide with the end of the material to be amplified. PCR can be used to amplify specific RNA sequences, specific DNA sequences from total genomic DNA and cDNA sequences transcribed from total cellular RNA, phage, plasmid sequences, or the like. See generally Mullis, et al., (1987) Cold Spring Harbor Symp. Quant. Biol. 51: 263; Ed. Erlich (1989) PCR TECHNOLOGY (Stockton Press, N.Y.). The PCR used herein is considered to be an example, but not the only one, of a nucleic acid polymerase reaction method for amplifying a nucleic acid test sample, and the method comprises using known nucleic acids as primers and nucleic acid polymerases to amplify or produce a specific moiety of the nucleic acid.

"Isolated" refers to a purified state, and in this case means that the designated molecule is substantially free of other biomolecules, such as nucleic acids, proteins, lipids, carbohydrates, or other materials (such as cell debris and growth medium). Generally, the term "isolated" does not mean the complete absence of such substances or the absence of water, buffers or salts, unless they are present in amounts that will significantly interfere with the experimental or therapeutic use of the compounds described herein.

The term "optional" or "optionally" means that the event or circumstance subsequently described may, but not necessarily, occur, and that the description includes instances where the event or circumstance occurs or does not occur.

The term "pharmaceutical composition" refers to a mixture containing one or more of the compounds described herein or physiologically/pharmaceutically acceptable salts or prodrugs thereof, and other chemical components, and the other components are, for example, physiologically/pharmaceutically acceptable carriers and excipients. The purpose of the pharmaceutical composition is to promote the administration to an organism, which facilitates the absorption of the active ingredient, thereby exerting biological activity.

The term "pharmaceutically acceptable carrier" refers to any inactive substance suitable for use in formulations for delivery of an antibody or an antigen-binding fragment. The carrier can be an anti-adhesive agent, binder, coating, disintegrant, filler or diluent, preservative (such as antioxidant, antibacterial or antifungal agent), sweetener, absorption delaying agent, wetting agent, emulsifier, buffer, etc. Examples of suitable pharmaceutically acceptable carriers include water, ethanol, polyol (such as glycerol, propanediol and polyethylene glycol), dextrose, vegetable oil (such as olive oil), saline, buffer, buffered saline, and isotonic agent such as sugar, polyol, sorbitol and sodium chloride.

In addition, the present disclosure includes agents for treating diseases related to positive cells of the target antigen (e.g., ANGPTL3), and the agents comprise the anti-ANGPTL3 antibody or the antigen-binding fragment thereof of the present disclosure as an active ingredient.

There is no limitation for the diseases related to ANGPTL3 in the present disclosure, as long as it is a disease related to ANGPTL3. For example, the therapeutic response induced by the molecule of the present disclosure can be achieved by binding to human ANGPTL3 and then blocking the binding of ANGPTL3 to its receptors. Thus, the molecules of the present disclosure, when in preparations and formulations suitable for therapeutic applications, are very useful for those suffering from hypercholesterolemia, hyperlipidemia, an atherosclerotic disease, or the like.

In addition, the present disclosure relates to methods for immunodetection or determination of the target antigen (e.g., ANGPTL3), reagents for immunodetection or determination of the target antigen (e.g., ANGPTL3), methods for immunodetection or determination of cells expressing the target antigen (e.g., ANGPTL3) and diagnostic agents for diagnosing diseases related to positive cells of the target antigen (e.g., ANGPTL3), which includes the antibody or the antibody fragment of the present disclosure as an active ingredient, which specifically recognizes the target antigen (e.g., human ANGPTL3) and binds to the amino acid sequence of the extracellular region or a three-dimensional structure thereof.

In the present disclosure, the method used for detection or determination of the amount of the target antigen (e.g., ANGPTL3) may be any known method. For example, it includes immunodetection or determination methods.

The immunodetection or determination methods are methods of detecting or determining the amount of antibody or antigen using labeled antigens or antibodies. Examples of immunodetection or determination methods include radioimmunoassay (RIA), enzyme immunoassay (EIA or ELISA), fluorescence immunoassay (FIA), luminescence immunoassay, western blotting, physicochemical methods, etc.

The aforementioned diseases related to positive cells of ANGPTL3 can be diagnosed by detecting or determining cells expressing ANGPTL3 by using the antibody or the antibody fragment of the present disclosure.

In order to detect cells expressing the polypeptide, known immunodetection methods can be used, preferably immunoprecipitation, fluorescent cell staining, immunohistochemical staining, etc. In addition, fluorescent antibody staining method utilizing the FMAT8100HTS system (Applied Biosystem) can be used.

In the present disclosure, there is no particular limitation for the sample used for detection or determination of the target antigen (e.g., ANGPTL3), as long as it has the possibility of comprising cells expressing the target antigen (e.g., ANGPTL3), such as histocyte, blood, plasma, serum, pancreatic juice, urine, feces, tissue fluid or culture solution.

According to the required diagnostic method, the diagnostic agent containing the monoclonal antibody or the antibody fragment thereof of the present disclosure can also contain reagents for performing antigen-antibody reaction or reagents for detecting the reaction. The reagents for performing the antigen-antibody reaction include buffers, salts, etc. The reagents for detection include reagents commonly used in immunodetection or determination methods, for example labeled second antibodies that recognize the monoclonal antibody, the antibody fragment thereof or the conjugate thereof, and substrates corresponding to the label, etc.

One or more embodiments of the present invention are described in detail in the specification above. Although any methods and materials similar or identical to those described herein can also be used to implement or test the present invention, preferred methods and materials are described below. Other features, objects and advantages of the present invention will be apparent from the description and the claims. In the specification and claims, singular forms include plural referents unless otherwise indicated clearly in the context. Unless otherwise defined, all technical and scientific terms used herein have the meanings generally understood by those of ordinary skill in the art to which the present invention belongs. All the patents and publications cited in the specification are incorporated by reference. The following examples are set forth in order to more fully illustrate the preferred embodiments of the present invention. These examples should not be construed in any way as limiting the scope of the present invention, which is defined by the claims.

### BRIEF DESCRIPTION OF THE DRAWINGS

FIG. 1 shows the effect of the antibody P8BG on serum triglyceride of Sprague Dawley (SD) rats;
FIG. 2 shows the effect of antibody P3G on serum triglyceride of SD rats;
FIG. 3 shows the effect of anti-ANGPTL3 antibodies on plasma triglyceride of high-fat-diet (HFD) mice;
FIG. 4 shows the effect of anti-ANGPTL3 antibodies on plasma low-density lipoprotein cholesterol (LDL-C) of HFD mice;
FIG. 5 shows the effect of anti-ANGPTL3 antibodies on plasma total cholesterol of HFD mice;
FIG. 6 shows the effect of anti-ANGPTL3 antibodies on plasma triglyceride of APOE mice (apolipoprotein E (ApoE) gene knockout mice);
FIG. 7 shows the effect of anti-ANGPTL3 antibodies on plasma high-density liptein cholesterol (HDL-C) of APOE mice;
FIG. 8 shows the results of *in vivo* pharmacokinetic experiment of anti-ANGPTL3 antibodies in mice; and
FIG. 9 shows the results of *in vivo* pharmacokinetic experiment of anti-ANGPTL3 antibodies in cynomolgus monkeys.

### DETAILED DESCRIPTION

The following examples further illustrate the present disclosure, but the present disclosure is not limited thereto.

Experimental procedures without conditions specified in the Examples or Test Examples of the present disclosure are generally conducted according to conventional conditions, or according to conditions recommended by the manufacturer of the starting materials or commercial products. See Sambrook et al., Molecular Cloning: A Laboratory Manual, Cold Spring Harbor Laboratory; Ausubel et al., Current Protocols in Molecular Biology, Greene Publishing Associates and Wiley Interscience, NY. Reagents without specific origins indicated are commercially available conventional reagents.

### Examples

### Example 1: Design and Expression of ANGPTL3 Antigens

Human ANGPTL3 protein (Uniprot No.: Q9Y5C1), mouse ANGPTL3 protein (Uniprot No.: Q9R182), cynomolgus monkey ANGPTL3 protein (Uniprot No.: A0A2K5UDC5) and rat ANGPTL3 protein (Uniprot No.: F7FHP0) were used as templates of the ANGPTL3 of the present disclosure for designing antigens and proteins for detection related to the present disclosure. Different tags were fused to the ANGPTL3 proteins, and the obtained proteins were each subjected to cloning into a pTT5 vector (Biovector, Cat #: 102762), transient transfection expression in 293 cells and purification, thus obtaining the antigens and proteins for detection of the present disclosure.
1. Full-length human ANGPTL3: human-ANGPTL3(17-460)-His, used for immunization and detection and having the following amino acid sequence: 
MEFGLSWLFLVAILKGVQCSRIDQDNSSFDSLSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
ETPEHPEVTSLKTFVEKQDNSIKDLLQTVEDQYKQLNQQHSQIKEIENQLR
RTSIQEPTEISLSSKPRAPRTTPFLQLNEIRNVKHDGIPAECTTIYNRGEHTS
GMYAIRPSNSQVFHVYCDVISGSPWTLIQHRIDGSQNFNETWENYKYGFGR
LDGEFWLGLEKIYSIVKQSNYVLRIELEDWKDNKHYIEYSFYLGNHETNYT
LHLVAITGNVPNAIPENKDLVFSTWDHKAKGHFNCPEGYSGGWWWHDEC
GENNLNGKYNKPRAKSKPERRRGLSWKSQNGRLYSIKSTKMLIHPTDSESF
EHHHHHHHHHH
SEQ ID NO: 1
 Note: the single-underlined part represents signal peptide sequence, the italicized part represents the His sequence, and the bold part represents the amino acids of positions 17 to 460 of the full-length human ANGPTL3 protein.
2. Extracellular region of human ANGPTL3: human ANGPTL3(17-170)-Flag-His, used for immunization and detection and having the following amino acid sequence: 
MEFGLSWLFLVAILKGVQCSRIDQDNSSFDSLSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
ETPEHPEVTSLKTFVEKGSSDYKDDDDKHHHHHH
SEQ ID NO: 2
 Note: the single-underlined part represents the signal peptide, the double-underlined part represents the linker peptide, the italicized part represents the Flag and His, and the bold part represents the amino acids of positions 17 to 170 of the full-length human ANGPTL3 protein.
3. Fusion protein of the extracellular region of human ANGPTL3 (human ANGPTL3(17-170)) and the mouse IgG2aFc fragment (abbreviated as mFc): human ANGPTL3(17-170)-mFc, used for detection and having the following amino acid sequence: 
MEFGLSWLFLVAILKGVQCSRIDQDNSSFDSLSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
ETPEHPEVTSLKTFVEKEPRGPTIKPCPPCKCPAPNLLGGPSVFIFPPKIKDVLMI
SLSPIVTCVVVDVSEDDPDVQISWFVNNVEVHTAQTQTHREDYNSTLRVVSALPIQHQ
DWMSGKEFKCKVNNKDLPAPIERTISKPKGSVRAPQVYVLPPPEEEMTKKQVTLTC
MVTDFMPEDIYVEWTNNGKTELNYKNTEPVLDSDGSYFMYSKLRVEKKNWVERNSY
SCSVVHEGLHNHHTTKSFSRTPGK
SEQ ID NO: 3
 Note: the underlined part represents the signal peptide, the italicized part represents the mouse IgG2aFc, and the bold part represents the amino acids of positions 17 to 170 of the full-length human ANGPTL3 protein.
4. Fusion protein of the extracellular region of human ANGPTL3 (human ANGPTL3(17-220)) and the mouse IgG2aFc fragment: human ANGPTL3(17-220)-mFc, which can be used for immunization and detection, and has the following amino acid sequence: 
MEFGLSWLFLVAILKGVQCSRIDQDNSSFDSLSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
ETPEHPEVTSLKTFVEKQDNSIKDLLQTVEDQYKQLNQQHSQIKEIENQLR
RTSIQEPTEISLSSKPEPRGPTIKPCPPCKCPAPNLLGGPSVFIFPPKIKDVLMISLS
PIVTCVVVDVSEDDPDVQISWFVNNVEVHTAQTQTHREDYNSTLRVVSALPIQHQDW
MSGKEFKCKVNNKDLPAPIERTISKPKGSVRAPQVYVLPPPEEEMTKKQVTLTCMVT
DFMPEDIYVEWTNNGKTELNYKNTEPVLDSDGSYFMYSKLRVEKKNWVERNSYSCS
VVHEGLHNHHTTKSFSRTPGK
SEQ ID NO: 4
 Note: the underlined part represents the signal peptide, the italicized part represents the mouse IgG2aFc, and the bold part represents the amino acids of positions 17 to 220 of the full-length human ANGPTL3 protein.
5. Full-length mouse ANGPTL3: mouse ANGPTL3(17-455)-His, used for immunization and detection and having the following amino acid sequence: 
MEFGLSWLFLVAILKGVQCSRVDPDLSSFDSAPSEPKSRFAMLDDVKILANGL
LQLGHGLKDFVHKTKGQINDIFQKLNIFDQSFYDLSLRTNEIKEEEKELRR
TTSTLQVKNEEVKNMSVELNSKLESLLEEKTALQHKVRALEEQLTNLILSP
 
AGAQEHPEVTSLKSFVEQQDNSIRELLQSVEEQYKQLSQQHMQIKEIEKQL
RKTGIQEPSENSLSSKSRAPRTTPPLQLNETENTEQDDLPADCSAVYNRGEH
TSGVYTIKPRNSQGFNVYCDTQSGSPWTLIQHRKDGSQDFNETWENYEKG
FGRLDGEFWLGLEKIYAIVQQSNYILRLELQDWKDSKHYVEYSFHLGSHET
NYTLHVAEIAGNIPGALPEHTDLMFSTWNHRAKGQLYCPESYSGGWWWN
DICGENNLNGKYNKPRTKSRPERRRGIYWRPQSRKLYAIKSSKMMLQPTT
HHHHHHHHHH
SEQ ID NO: 5
 Note: the underlined part represents signal peptide, the italicized part represents the His sequence, and the bold part represents the amino acids of positions 17 to 455 of the full-length mouse ANGPTL3 protein.
6. Fusion protein of the extracellular region of mouse ANGPTL3 (mouse-ANGPTL3(17-220)) and mouse IgG2aFc segment: mouse ANGPTL3(17-220)-mFc, used for immunization and having the following amino acid sequence: 
MEFGLSWLFLVAILKGVQCSRVDPDLSSFDSAPSEPKSRFAMLDDVKILANGL
LQLGHGLKDFVHKTKGQINDIFQKLNIFDQSFYDLSLRTNEIKEEEKELRR
TTSTLQVKNEEVKNMSVELNSKLESLLEEKTALQHKVRALEEQLTNLILSP
AGAQEHPEVTSLKSFVEQQDNSIRELLQSVEEQYKQLSQQHMQIKEIEKQL
RKTGIQEPSENSLSSKSEPRGPTIKPCPPCKCPAPNLLGGPSVFIFPPKIKDVLMIS
LSPIVTCVVVDVSEDDPDVQISWFVNNVEVHTAQTQTHREDYNSTLRVVSALPIQHQ
DWMSGKEFKCKVNNKDLPAPIERTISKPKGSVRAPQVYVLPPPEEEMTKKQVTLTC
MVTDFMPEDIYVEWTNNGKTELNYKNTEPVLDSDGSYFMYSKLRVEKKNWVERNSY
SCSVVHEGLHNHHTTKSFSRTPGK
SEQ ID NO: 6
 Note: the underlined part represents the signal peptide, the italicized part represents the mouse IgG2aFc, and the bold part represents the amino acids of positions 17 to 220 of the full-length mouse ANGPTL3 protein.
7. Full-length cynomolgus monkey ANGPTL3: cynomolgus monkey ANGPTL3(17-460)-His, used for detection and having the following amino acid sequence: 
MEFGLSWLFLVAILKGVQCSRIDQDNSSFDSVSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
ATPEHPEVTSLKSFVEKQDNSIKDLLQTVEEQYKQLNQQHSQIKEIENQLR
MTNIQEPTEISLSSKPRAPRTTPFLQLNEIRNVKHDGIPADCTTIYNRGEHIS
GTYAIRPSNSQVFHVYCDVVSGSPWTLIQHRIDGSQNFNETWENYKYGFGR
LDGEFWLGLEKIYSIVKQSNYVLRIELEDWKDNKHYIEYSFYLGNHETNYT
 
LHVVKITGNVPNAIPENKDLVFSTWDHKAKGHFSCPESYSGGWWWHDEC
GENNLNGKYNKPRTKSKPERRRGLSWKSQNGRLYSIKSTKMLIHPTDSESF
EHHHHHHHHHH
SEQ ID NO: 7
 Note: the underlined part represents the signal peptide, the italicized part represents the His sequence, and the bold part represents the amino acids of positions 17 to 460 of the full-length cynomolgus monkey ANGPTL3 protein.
8. Full-length rat ANGPTL3: rat ANGPTL3(17-455)-His, used for detection and having the following amino acid sequence: 
MEFGLSWLFLVAILKGVQCSRVDPDLSPFDSVPSEPKSRFAMLDDVKILANG
LLQLGHGLKDFVHKTKGQINDIFQKLNIFDQCFYDLSLQTNEIKEEEKELR
RTTSKLQVKNEEVKNMSLELNSKLESLLEEKMALQHRVRALEEQLTSLVQ
NPPGAREHPEVTSLKSFVEQQDNSIRELLQSVEEQYKQLSQQHIQIKEIENQ
LRKTGIQEPTENSLYSKPRAPRTTPPLHLKEAKNIEQDDLPADCSAIYNRGE
HTSGVYTIRPSSSQVFNVYCDTQSGTPRTLIQHRKDGSQNFNQTWENYEKG
FGRLDGEFWLGLEKIYAIVKQSNYILRLELQDWKDSKHYAEYSFHLGNHE
TNYTLHVAEIAANIPEALPEHRDLMFSTWDHRAKGQLYCPESYSGGWWFS
DMCGENNLNGKYNKPRAKSKPERRRGISWRPRGGKLYSIKSSKMMLQPT
THHHHHHHHHH
SEQ ID NO: 8
 Note: the underlined part represents the signal peptide, the italicized part represents the His sequence, and the bold part represents the amino acids of positions 17 to 455 of the full-length rat ANGPTL3 protein.

### Example 2: Purification of ANGPTL3 Antigen Proteins, Hybridoma Antibodies and Recombinant Antibodies

### 1. Purification of recombinant proteins with His-tag or recombinant proteins with Flag-His-tag

A cell expression sample was centrifuged at a high speed to remove cells and insoluble impurities, the supernatant was collected, and imidazole was added to reach a final concentration of 5 mM. A nickel column was equilibrated with a PBS solution containing 5 mM imidazole and washed with 2-5 column volumes. Cell supernatant was then loaded onto the column. The column was washed with PBS solution containing 5 mM imidazole until A₂₈₀ reading dropped to baseline. The chromatography column was then washed with a mixture of PBS and 10 mM imidazole to remove non-specifically bound impure proteins, and the flow-through was collected. The target protein was eluted with PBS solution containing 300 mM imidazole and the elution peak was collected.

The collected eluate was further purified using a Superdex gel filtration column and then collected with tubes. The obtained sample was identified by SDS-PAGE, peptide map and LC-MS, and then aliquoted for later use if it was determined to be correct. Thus, the recombinant proteins with His tag or the recombinant proteins with Flag-His tag were obtained.

### 2. Purification of hybridoma antibodies or recombinant proteins with mFc tag

A sample was centrifuged at a high speed to remove cells and insoluble impurities, and the supernatant was retained. A Protein A affinity column was equilibrated with 1× PBS and washed with 2-5 column volumes. The centrifuged cell expression supernatant sample was loaded onto the column. The column was washed with 1× PBS until A₂₈₀ reading dropped to baseline. The column was then washed with 1× PBS. The target protein was eluted with 0.1 M acetic acid (pH 3.5-4.0) and collected, and then the pH of the eluted protein was immediately adjusted to neutral with 1 M Tris-HCl (pH 7.0), and finally the eluted protein was subjected to dialysis or buffer exchange to 1× PBS buffer. The sample was collected, identified by electrophoresis, peptide map and LC-MS, and then aliquoted for later use if it was determined to be correct. Thus, the recombinant proteins with mFc tag or the hybridoma antibodies were obtained.

### 3. Purification of antibodies and recombinant proteins with Fc tag

A sample was centrifuged at a high speed to remove cells and insoluble impurities, and the supernatant was retained. A Protein G affinity column was equilibrated with 1× PBS and washed with 2-5 column volumes. The centrifuged cell expression supernatant sample was loaded onto the column. The column was washed with 1× PBS until A₂₈₀ reading dropped to baseline. The column was then washed with 1× PBS. The target protein was eluted with acetic acid (pH 3.0) and collected, and then the pH of the eluted protein was immediately adjusted to neutral with 1 M Tris-HCl (pH 7.0), and finally the eluted protein was subjected to dialysis or buffer exchange to 1× PBS buffer. The sample was collected, identified by electrophoresis, peptide map and LC-MS, and then aliquoted for later use if it was determined to be correct. Thus, the antibody or the recombinant protein with hFc tag was obtained.

### Example 3: Preparation of Anti-ANGPTL3 Antibodies

### 1. Screening of anti-ANGPTL3 human-derived antibodies by phage library

A human phage single-chain antibody library was packaged and concentrated, and then the phage library (10¹²-10¹³/pfu) was suspended in 1 mL of 2% MPBS (PBS containing 2% of skim milk powder), and then 100 µL of Dynabeads^{®} M-280 streptavidin (Invitrogen, Cat No. 11206D) was added. The tube was placed on a rotating plate and rolled over repeatedly, and then blocked at room temperature for 1 h. The tube was then placed on a magnetic rack for 2 min, the Dynabeads were removed, and then the phage library was transferred to a new tube. 2 µg/mL biotin-labeled human ANGPTL3(17-460)-His was added to the blocked phage library, and then the tube was placed on the rotating plate and rolled over repeatedly for 1 h. At the same time, 100 µL of Dynabeads was suspended in 1 mL of 2% MPBS, and then the tube was placed on a rotating plate and rolled over repeatedly, and then blocked at room temperature for 1 h. The tube was then placed on a magnetic rack for 2 min, and the blocking solution was pipetted off. The blocked Dynabeads were added to the mixture of the phage library and human ANGPTL3(17-460)-His, and the tube containing the resulting mixture was placed on a rotating plate and rolled over repeatedly for 15 min. The tube was then placed on a magnetic rack for 2 min, and the mixed solution was pipetted off. Dynabeads were washed 10 times with 1 mL of PBST (PBS containing 0.1% Tween-20), and 0.5 mL of 1 mg/mL trypsin (Sigma, Cat No. T1426-250MG) was added. The tube was then placed on a rotating plate and incubated for 15 min by rolling over repeatedly, and then elution was performed. The eluted phage was then used to infect E. coli TG1, which was then used for plating. Single clones were randomly picked for phage ELISA.

The clones were seeded into a 96-well deep-well plate (Nunc Cat No. 260251) and incubated at 37 °C for 16-18 h. A small number of the incubated clones were inoculated into another 96-well deep-well plate until the OD₆₀₀ reached about 0.5, and M13K07 helper phage (NEB, Cat No. N0315S) was then added for packaging. The mixture was centrifuged at 4000 g for 10 min to remove bacteria, and the culture solution was pipetted out for ELISA detection of ANGPTL3 binding. The positive clone strains were cryopreserved and sent to a sequencing company for sequencing. The amino acid sequences corresponding to the positive clones P3 and P8 are as follows:
> Heavy chain variable region sequence of P3: 
QVQLVQSGAEVKKPGASVKVSCKSSGYTFTDYYLHWLRQAPGQGPEWMGWISPNS
GGTSYAQKFQGRVTMTRDTSISTAYMELSSLRSDDTAVYYCARDIATLGNFFTYGM
DVWGQGTTVTVSS
SEQ ID NO: 9
> Light chain variable region sequence of P3: 
DIQLTQSPSSLSASVGDRVTITCRASQGIRNDLGWYQQKPGKAPKLLIYAASSLQSG
VPSRFSGSGSGTDFTLTISSLQPEDFATYYCQQSYSSWTFGQGTKVEIK
SEQ ID NO: 10
> Heavy chain variable region sequence of P8: 
QVQLVQSGAEVKKPGASVKVSCKASGYTFTSYDINWVRQAPGQGLEWVGLINPRD
DSTSYAQKFQGRVTMTRDTSTSTMYMELSSLRSEDTAVYFCARDLGSIREVLYYGM
DVWGQGTTVTVSS
SEQ ID NO: 11
> Light chain variable region sequence of P8: 
DVVMTQSPLSLPVTPGEPASISCRSSQSLLHSNGYTYLDWYLQKPGQSPQLLIYLGS
NRASGVPDRFSGSGSGTDFTLKISRVEAEDVGVYYCMQALQTPLTFGQGTRLEIK
SEQ ID NO: 12
 Note: the underlined parts in the sequences represent the CDR sequences determined according to the Kabat numbering system, and the italicized parts represent FR sequences.

### 2. Mutation of anti-ANGPTL3 antibody

Based on the three-dimensional structure of the P8 antibody, the amino acid residue at position 55 (naturally sequentially numbered) in the heavy chain variable region of P8 was mutated from D to E, and the amino acid residue at position 34 (naturally sequentially numbered) in the light chain variable region of P8 was mutated from G to V, thus obtaining a novel antibody molecule P8B, wherein the amino acid sequence of P8B is as follows:
> Heavy chain variable region sequence of P8B: 
QVQLVQSGAEVKKPGASVKVSCKASGYTFTSYDINWVRQAPGQGLEWVGLINPRE
DSTSYAQKFQGRVTMTRDTSTSTMYMELSSLRSEDTAVYFCARDLGSIREVLYYGM
DVWGQGTTVTVSS
SEQ ID NO: 13
> Light chain variable region sequence of P8B: 
DVVMTQSPLSLPVTPGEPASISCRSSQSLLHSNVYTYLDWYLQKPGQSPQLLIYLGS
NRASGVPDRFSGSGSGTDFTLKISRVEAEDVGVYYCMQALQTPLTFGQGTRLEIK
SEQ ID NO: 14
Note: the underlined parts in the sequences represent the CDR sequences determined according to the Kabat numbering system, and the italicized parts represent FR sequences.

**Table 2. CDR sequences of anti-ANGPTL3 antibody molecules**

| Antibody molecule | Light chain CDR | | Heavy chain CDR | |
|---|---|---|---|---|
| P3 | LCDR1 | RASQGIRNDLG (SEQ ID NO: 15) | HCDR1 | DYYLH (SEQ ID NO: 18) |
| | LCDR2 | AASSLQS (SEQ ID NO: 16) | HCDR2 | WISPNSGGTSYAQKFQG (SEQ ID NO: 19) |
| | LCDR3 | QQSYSSWT (SEQ ID NO: 17) | HCDR3 | DIATLGNFFTYGMDV (SEQ ID NO: 20) |
| P8 | LCDR1 | RSSQSLLHSNGYTYLD (SEQ ID NO: 21) | HCDR1 | SYDIN (SEQ ID NO: 24) |
| | LCDR2 | LGSNRAS (SEQ ID NO: 22) | HCDR2 | LINPRDDSTSYAQKFQG (SEQ ID NO: 25) |
| | LCDR3 | MQALQTPLT (SEQ ID NO: 23) | HCDR3 | DLGSIREVLYYGMDV (SEQ ID NO: 26) |
| P8B | LCDR1 | RSSQSLLHSNVYTYLD (SEQ ID NO: 27) | HCDR1 | SYDIN (SEQ ID NO: 24) |
| | LCDR2 | LGSNRAS (SEQ ID NO: 22) | HCDR2 | LINPREDSTSYAQKFQG (SEQ ID NO: 28) |
| | LCDR3 | MQALQTPLT (SEQ ID NO: 23) | HCDR3 | DLGSIREVLYYGMDV (SEQ ID NO: 26) |

### 3. Construction and expression of anti-ANGPTL3 antibodies

A primer was designed, and then a VH/VK gene fragment of the antibody was constructed by PCR and subjected to homologous recombination with an expression vector pHr (with a signal peptide and a constant region gene (CH1-FC/CL) fragment), thus constructing an expression vector VH-CH1-FC-pHr/VK-CL-pHr for a full-length antibody. The heavy chain constant region of the antibody may be selected from the group consisting of constant regions of human IgG1, IgG2, IgG4 and variants thereof, and the light chain constant region may be selected from the group consisting of the light chain constant regions of human κ and λ chains or variants thereof. Illustratively, the antibody heavy chain constant region is selected from the human IgG4-YTE variant having a sequence set forth in SEQ ID NO: 29, and the light chain constant region is selected from the constant region of human κ chain having a sequence set forth in SEQ ID NO: 30.

Heavy chain constant region sequence of human IgG4-YTE variant:

ASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPP
CPAPEFLGGPSVFLFPPKPKDTLYITREPEVTCVVVDVSQEDPEVQFNWYVDGV
EVHNAKTKPREEQFNSTYRVVSVLTVLHQDWLNGKEYKCKVSNKGLPSSIEKT
ISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLS
LSLGK
SEQ ID NO: 29

Light chain constant region sequence of human κ chain:



SEQ ID NO: 30

Illustratively, the light/heavy chain constant regions described above, together with the variable regions of P3, P8 and P8B described above, form complete anti-ANGPTL3 antibodies: P3G, P8G and P8BG, and the light/heavy chain sequences of the antibodies are as follows:
>Heavy chain sequence of P3G: 
QVQLVQSGAEVKKPGASVKVSCKSSGYTFTDYYLHWLRQAPGQGPEWMGWISPNS
GGTSYAQKFQGRVTMTRDTSISTAYMELSSLRSDDTAVYYCARDIATLGNFFTYGM
DVWGQGTTVTVSSASTKGPSVFPLAPCSRSTSESTAALGCLVKDYFPEPVTVSWN
SGALTSGVHTFPAVLQSSGLYSLSSVVTVPSSSLGTKTYTCNVDHKPSNTKVDK
RVESKYGPPCPPCPAPEFLGGPSVFLFPPKPKDTLYITREPEVTCVVVDVSQEDP
EVQFNWYVDGVEVHNAKTKPREEQFNSTYRVVSVLTVLHQDWLNGKEYKCK
 
VSNKGLPSSIEKTISKAKGQPREPQVYTLPPSQEEMTKNQVSLTCLVKGFYPSDI
AVEWESNGQPENNYKTTPPVLDSDGSFFLYSRLTVDKSRWQEGNVFSCSVMHE
ALHNHYTQKSLSLSLGK
SEQ ID NO: 31
>Light chain sequence of P3G: 
DIQLTQSPSSLSASVGDRVTITCRASQGIRNDLGWYQQKPGKAPKLLIYAASSLQSG
VPSRFSGSGSGTDFTLTISSLQPEDFATYYCQQSYSSWTFGQGTKVEIKRTVAAPSV
FIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID NO: 32
>Heavy chain sequence of P8G: 
QVQLVQSGAEVKKPGASVKVSCKASGYTFTSYDINWVRQAPGQGLEWVGLINPRD
DSTSYAQKFQGRVTMTRDTSTSTMYMELSSLRSEDTAVYFCARDLGSIREVLYYGM
DVWGQGTTVTVSSASTKGPSVFPLAPCSRSTSESTAALGCLVKDYFPEPVTVSWN
SGALTSGVHTFPAVLQSSGLYSLSSVVTVPSSSLGTKTYTCNVDHKPSNTKVDK
RVESKYGPPCPPCPAPEFLGGPSVFLFPPKPKDTLYITREPEVTCVVVDVSQEDP
EVQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSDI
AVEWESNGQPENNYKTTPPVLDSDGSFFLYSRLTVDKSRWQEGNVFSCSVMHE
ALHNHYTQKSLSLSLGK
SEQ ID NO: 33
>Light chain sequence of P8G: 
DVVMTQSPLSLPVTPGEPASISCRSSQSLLHSNGYTYLDWYLQKPGQSPQLLIYLGS
NRASGVPDRFSGSGSGTDFTLKISRVEAEDVGVYYCMQALQTPLTFGQGTRLEIKR
TVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 34
>Heavy chain sequence of P8BG: 
QVQLVQSGAEVKKPGASVKVSCKASGYTFTSYDINWVRQAPGQGLEWVGLINPRE
DSTSYAQKFQGRVTMTRDTSTSTMYMELSSLRSEDTAVYFCARDLGSIREVLYYGM
DVWGQGTTVTVSSASTKGPSVFPLAPCSRSTSESTAALGCLVKDYFPEPVTVSWN
SGALTSGVHTFPAVLQSSGLYSLSSVVTVPSSSLGTKTYTCNVDHKPSNTKVDK
RVESKYGPPCPPCPAPEFLGGPSVFLFPPKPKDTLYITREPEVTCVVVDVSQEDP
EVQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSDI
AVEWESNGQPENNYKTTPPVLDSDGSFFLYSRLTVDKSRWQEGNVFSCSVMHE
ALHNHYTQKSLSLSLGK
SEQ ID NO: 35
>Light chain sequence of P8BG:

DVVMTQSPLSLPVTPGEPASISCRSSQSLLHSNVYTYLDWYLQKPGQSPQLLIYLGS
NRASGVPDRFSGSGSGTDFTLKISRVEAEDVGVYYCMQALQTPLTFGQGTRLEIKR
TVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 36

### Verification of activity of the antibodies of the present disclosure with biochemical test methods

### Test Example 1: ELISA Assay for Binding of Anti-ANGPTL3 Antibodies to ANGPTL3 Proteins

An anti-ANGPTL3 antibody inhibited or interfered with at least one activity of an ANGPTL3 protein by binding to the ANGPTL3 protein. The binding activity of the anti-ANGPTL3 antibody to the ANGPTL3 antigen was tested by an ELISA assay. The ANGPTL3 protein (human ANGPTL3(17-170)-mFc) was immobilized on a 96-well microplate by binding to the goat anti-mouse IgG coated on the microplate, and the binding activity of the antibody to the ANGPTL3 was determined according to the intensity of the signal after the antibody was added. Meanwhile, the ANGPTL3 proteins with a HIS tag (mouse ANGPTL3(17-455)-His, cynomolgus monkey ANGPTL3(17-460)-His and rat ANGPTL3(17-455)-His) labelled with a biotin labeling kit (Dojindo China, LK03) were immobilized on a 96-well microplate by binding to streptavidin coated on the microplate, and the binding activity of the antibody to the ANGPTL3 was determined according to the intensity of the signal after the antibody was added.

Goat anti-mouse IgG (Sigma, M3534-1ML) was diluted to a concentration of 2 µg/mL with PBS buffer (Shanghai BasalMedia, B320, pH 7.4), added to a 96-well microplate (Corning, CLS3590-100EA) at 50 µL/well, and then incubated in an incubator at 37 °C for 3 h or incubated at 4 °C overnight (16-18 h). After the liquid was discarded, a PBS-diluted 5% skim milk (BD, 232100) blocking solution was added at 250 µL/well, and the mixture was incubated in an incubator at 37 °C for 3 h or incubated at 4 °C overnight (16-18 h) for blocking. After the blocking was finished, the blocking solution was discarded and the plate was washed 3 times with PBST buffer (pH 7.4 PBS containing 0.05% Tween-20), and then human ANGPTL3(17-170)-mFc (with sequence set forth in SEQ ID NO: 3) diluted to 0.5 µg/mL with a sample diluent (pH 7.4 PBS containing 1% BSA) was added at 50 µL/well. The mixture was incubated in an incubator at 37 °C for 2 h. After the incubation was finished, the reaction solution in the microplate was discarded. The plate was washed 3 times with PBST, and then a test antibody that was diluted with a sample diluent to different concentrations was added at 50 µL/well. The mixture was incubated in an incubator at 37 °C for 2 h. After the incubation was finished, the plate was washed 3 times with PBST, and a goat anti-human secondary antibody (Jackson Immuno Research, 109-035-003) that was diluted with the sample diluent was added at 50 µL/well. The mixture was incubated at 37 °C for 1 h. The plate was washed 3 times with PBST, and then TMB chromogenic substrate (KPL, 52-00-03) was added at 50 µL/well. The mixture was incubated at room temperature for 2-5 min, and the reaction was terminated by adding 1 M H₂SO₄ at 50 µL/well. The absorbance at 450 nm was read using a VersaMax microplate reader, and the binding EC₅₀ value of the anti-ANGPTL3 antibody to ANGPTL3 antigen protein was calculated. The experimental results are shown in Table 3.

Streptavidin (Sigma, S4762-5MG) was diluted to a concentration of 3 µg/mL with PBS buffer (Shanghai BasalMedia, B320, pH 7.4), added to a 96-well microplate (Corning, CLS3590-100EA) at 50 µL/well, and then incubated in an incubator at 37 °C for 3 h or incubated at 4 °C overnight (16-18 h). After the liquid was discarded, a PBS-diluted 5% skim milk (BD, 232100) blocking solution was added at 250 µL/well, and the mixture was incubated in an incubator at 37 °C for 3 h or incubated at 4 °C overnight (16-18 h) for blocking. After the blocking was finished, the blocking solution was discarded and the plate was washed 3 times with PBST buffer (pH 7.4 PBS containing 0.05% Tween-20), and biotin-labeled mouse ANGPTL3(17-455)-His (with sequence set forth in SEQ ID NO: 5), cynomolgus monkey ANGPTL3(17-460)-His (with sequence set forth in SEQ ID NO: 7) and rat ANGPTL3(17-455)-His (with sequence set forth in SEQ ID NO: 8) each diluted to 0.5 µg/mL with a sample diluent (pH 7.4 PBS containing 1% BSA) was added at 50 µL/well. The mixture was incubated in an incubator at 37 °C for 2 h. After the incubation was finished, the reaction solution in the microplate was discarded. The plate was washed 3 times with PBST, and then a test antibody that was diluted with a sample diluent to different concentrations was added at 50 µL/well. The mixture was incubated in an incubator at 37 °C for 2 h. After the incubation was finished, the plate was washed 3 times with PBST, and an HRP-labeled goat anti-mouse secondary antibody (Jackson Immuno Research, 115-035-003), a goat anti-human secondary antibody (Jackson Immuno Research, 109-035-003) or a mouse HRP/anti-M13 conjugate secondary antibody (Sino Biological, Cat No. 11973-MM05-100, used for phage screening) diluted with a sample diluent was added at 50 µL/well. The mixture was incubated at 37 °C for 1 h. The plate was washed 3 times with PBST, and then TMB chromogenic substrate (KPL, 52-00-03) was added at 50 µL/well. The mixture was incubated at room temperature for 2-5 min, and the reaction was terminated by adding 1 M H₂SO₄ at 50 µL/well. The absorbance at 450 nm was read using a VersaMax microplate reader, and the binding EC₅₀ value of the anti-ANGPTL3 antibody to ANGPTL3 antigen protein was calculated. The experimental results are shown in Table 3. The results show that the anti-ANGPTL3 antibodies of the present disclosure are capable of binding to ANGPTL3 proteins of different species.

**Table 3. ELISA assay for binding of anti-ANGPTL3 antibodies to ANGPTL3 proteins of different species**

| Antibodies | Binding to human ANGPTL3 EC50 (nM) | Binding to mouse ANGPTL3 EC50 (nM) | Binding to cynomolgus monkey ANGPTL3 EC50( nM) | Binding to rat ANGPTL3 EC50 (nM) |
|---|---|---|---|---|
| P3G | 2.480 | 1.631 | 4.555 | 1.441 |
| P8G | 0.361 | 1.732 | 2.528 | 2.115 |
| P8BG | 0.172 | 0.105 | 0.200 | 0.528 |

### Test Example 2: HTRF Assay for Binding of Anti-ANGPTL3 Antibodies to ANGPTL3 Antigen Proteins

HTRF assay was also used to detect the binding activity of the anti-ANGPTL3 antibody to the antigen. The ANGPTL3 with a HIS tag (human ANGPTL3(17-460)-His, mouse ANGPTL3(17-455)-His, cynomolgus monkey ANGPTL3(17-460)-His and rat ANGPTL3(17-455)-His) labelled with a biotin labeling kit (Dojindo China, LK03) bound to the HTRF reagent Streptavidin-Tb cryptata (Cisbio, 610SATLA), and after the antibody was added, the antibody bound to the HTRF reagent Pab anti-mouse IgG-XL665 (Cisbio, 61PAMXLA) and angiopoietin-like protein 3. The intensity of the signal was used to determine the binding activity of the antibody to the angiopoietin-like protein 3.

4 µg/mL biotin-labeled human ANGPTL3(17-460)-His (with sequence set forth in SEQ ID NO: 1), mouse ANGPTL3(17-455)-His (with sequence set forth in SEQ ID NO: 5), cynomolgus monkey ANGPTL3(17-460)-His (with sequence set forth in SEQ ID NO: 7) and rat ANGPTL3(17-455)-His (with sequence set forth in SEQ ID NO: 8) were each prepared using a diluent (pH 7.4 PBS containing 1% BSA)and added to a 384-well plate (PerkinElmer, optiplatte-384) at 5 µL/well, a mixed solution of the HTRF reagent Streptavidin-Tb cryptata and Pab anti-mouse IgG-XL665 was then added at 5 µL/well, and finally a test antibody that was diluted with a sample diluent to different concentrations was added at 10 µL/well. The mixture was incubated at 25 °C for 1 h. Values were read using a PHERAstar FS (BMG LabTECH) at the HTRF module, and the EC₅₀ value for binding of the anti-ANGPTL3 antibody to angiopoietin-like protein 3 was calculated. The experimental results are shown in Table 4. The results show that the antibodies of the present disclosure all have good binding activity for the ANGPTL3 antigens of human, mouse, cynomolgus monkey, rat and other species.

**Table 4. HTRF assay for binding of anti-ANGPTL3 antibodies to ANGPTL3 proteins**

| Antibodies | EC₅₀ (nM) for binding to ANGPTL3 proteins | | | |
|---|---|---|---|---|
| | Human ANGPTL3 | Mouse ANGPTL3 | Cynomolgus monkey ANGPTL3 | Rat ANGPTL3 |
| | | | | |
| P3G | 0.929 | 0.638 | 0.502 | 0.597 |
| P8G | 0.784 | 0.450 | 0.303 | 0.484 |
| P8BG | 0.277 | 0.321 | 0.360 | 0.362 |

### Test Example 3: Enzyme Activity Assay for Inhibition of LPL by Anti-ANGPTL3 Antibodies

The enzyme activity detection experiment of LPL was used for detecting the activity of anti-ANGPTL3 antibody in blocking the inhibition of LPL enzyme by ANGPTL3 protein.

Bovine LPL (Sigma, L2254-5KU) was diluted with a dilution buffer (pH 7.4 PBS + 2 mg/mL BSA) to 12.5 units and added to a 96-well plate (Corning, 3603) at 25 µL/well, then a test antibody that was diluted with a sample diluent to different concentrations was added at 25 µL/well., and then 27.6 µg/mL ANGPTL3 protein (human ANGPTL3(17-170) -Flag-His (with sequence set forth in SEQ ID NO: 2), mouse ANGPTL3(17-455)-His (with sequence set forth in SEQ ID NO: 5), cynomolgus monkey ANGPTL3(17-460)-His (with sequence set forth in SEQ ID NO: 7) and rat ANGPTL3(17-455)-His (with sequence set forth in SEQ ID NO: 8)) was added at 25 µL/well. The 96-well plate was shaken on a shaker for mixing the mixture well, which was then incubated in an incubator at 37 °C for 30 min. Finally, a 20 µM substrate (Sigma, 30058-10MG-F) diluted with a dilution buffer was added at 25 µL/well, and the 96-well plate was shaken on a shaker for mixing the mixture well, which was then incubated in an incubator at 37 °C for 30 min. The Ex535/Em612 was read using a Flexstation3 microplate reader. The concentrations and corresponding fluorescence data were processed using Graphpad Prism 5 software, and the IC₅₀ was calculated. The experimental results are shown in Table 5. The results show that the antibodies of the present disclosure exhibit good inhibition effect against LPL enzyme.

**Table 5. Assay Results of activity of anti-ANGPTL3 antibodies in blocking inhibition of LPL by ANGPTL3 of different species**

| Antibodies | IC50(nM) | | | |
|---|---|---|---|---|
| | Human ANGPTL3 | Mouse ANGPTL3 | Cyno ANGPTL3 | Rat ANGPTL3 |
| P3G | 68.9 | 167.5 | 42.4 | 66.5 |
| P8BG | 75.3 | 41.5 | 53.6 | 132.2 |

### Test example 4: Biacore Assay of Anti-ANGPTL3 Antibodies and ANGPTL3 Proteins

Biacore was used to determine the affinity of the test anti-ANGPTL3 antibodies for human, monkey, rat and mouse ANGPTL3, and the method is as follows:
A certain amount of test antibody was subjected to affinity capture by a Protein A biosensor chip (Cat. # 29127556, GE), and then the human, monkey, rat and mouse ANGPTL3 antigens (human-ANGPTL3 (R&D, 3829-AN), mouse ANGPTL3(17-455)-His (with a sequence set forth in SEQ ID NO: 5), cynomolgus monkey ANGPTL3(17-460)-His (with a sequence set forth in SEQ ID NO: 7) and rat ANGPTL3(17-455)-His (with a sequence set forth in SEQ ID NO: 8)) at a certain concentration each flowed through the surface of the chip. Reaction signals were detected in real time using a Biacore T200 instrument to obtain association and dissociation curves. After each cycle of dissociation was completed, the biochip was washed and regenerated using a glycine-hydrochloric acid regeneration solution (Cat. # BR-1003-54, GE, pH 1.5). The running buffer for the experiment was 1× HBS-EP buffer (Cat. # BR-1001-88, GE). The data obtained from the experiment were fitted with the (1:1) Langmuir model using GE Biacore T200 evaluation software (version 3.0) to obtain affinity values. The experimental results are shown in Table 6. The results show that the anti-ANGPTL3 antibodies of the present disclosure are capable of binding to the human, cynomolgus monkey, rat and mouse ANGPTL3 antigens with high affinity.

**Table 6. Results of Biacore assay of anti-ANGPTL3 antibodies and ANGPTL3 proteins**

| Antibodies | Antigens | Association constant (1/Ms) | Dissociation constant (1/s) | Affinity (M) |
|---|---|---|---|---|
| P3G | Human ANGPTL3 | 2.56E+06 | 7.31E-04 | 2.85E-10 |
| | Cynomolgus monkey ANGPTL3 | 4.15E+06 | 8.47E-04 | 2.04E-10 |
| | Mouse ANGPTL3 | 9.81E+05 | 8.27E-04 | 8.43E-10 |
| | Rat ANGPTL3 | 1.28E+06 | 8.20E-04 | 6.39E-10 |
| P8G | Human ANGPTL3 | 2.20E+06 | 3.33E-04 | 1.51E-10 |
| | Cynomolgus monkey ANGPTL3 | 8.37E+06 | 4.97E-04 | 5.93E-11 |
| | Mouse ANGPTL3 | 9.70E+05 | 3.45E-04 | 3.55E-10 |
| | Rat ANGPTL3 | 1.34E+06 | 3.35E-04 | 2.50E-10 |
| P8BG | Human ANGPTL3 | 6.14E+06 | 2.76E-04 | 4.50E-11 |
| | Cynomolgus monkey ANGPTL3 | 5.18E+06 | 3.98E-04 | 7.69E-11 |
| | Mouse ANGPTL3 | 1.46E+06 | 4.20E-04 | 2.88E-10 |
| | Rat ANGPTL3 | 1.21E+06 | 3.11E-04 | 2.57E-10 |

### Test Example 5: Evaluation of In Vivo Efficacy of Anti-ANGPTL3 Antibodies in Rats

### 1. In vivo lipid lowering experiment of antibody P8BG in rats

SD rats for experiments (aged 4 weeks, SPF, about 100 g, male, purchased from Shanghai Slac Laboratory Animal Co., Ltd.; certification No.: 20170005013017, SCXK (Shanghai) 2017-0005) were subjected to 1 week of adaptive feeding in the laboratory environment (in a 12/12 hour light/dark cycle, at a temperature of 23±1 °C and a humidity of 40-50%), and the animals were fed with standard sterile mouse feed and had free access to food and water. 7 days before the start of the experiment, the animals were fasted for 4 h, and then blood was collected from the orbit and centrifuged at 3500 RPM for 15 min, and serum was collected. Serum lipid concentration (triglyceride) was determined using the ADVIA 2400 chemical system (Siemens). The rats were divided into 6 groups by triglyceride level (non-relevant isotype human IgG protein (hIgG, the same below) as a negative control group, evinacumab (for sequence structure, see sequence of evinacumab in WHO Drug Information, Vol. 29, No. 3, 2015) 3.5 mpk group, evinacumab 7 mpk group, P8BG 3.5 mpk group, P8BG 1.75 mpk group, and P8BG 7 mpk group), 6 rats for each group. The rats in all the groups were each subjected to subcutaneous injection once, and then subjected to blood collection after 4 h of fasting on days 1, 5, 9, 13 and 16 after injection. Serum was separated out, and serum lipid concentration (triglyceride) was determined using the ADVIA 2400 chemical system (Siemens). The experimental data were expressed as Mean±standard deviation (Mean±SEM) and plotted using Graphpad Prism5 software, and TTEST was used for statistical analysis.

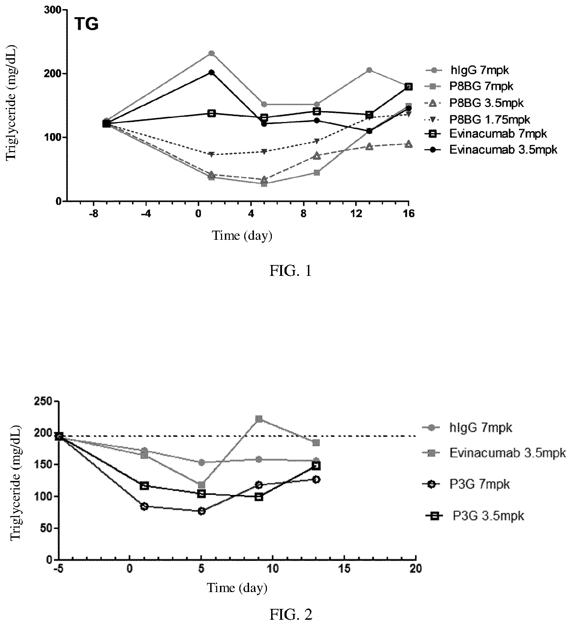

The experimental results are shown in FIG. 1 and Table 7. Compared with the negative control group, 1 day after the administration, triglyceride in each treatment group was decreased, and statistical differences were observed in the groups except for the evinacumab 3.5 mpk group. During the experiment, triglyceride in the P8BG 7 mpk group was decreased by 83.7% at maximum, triglyceride in the P8BG 3.5 mpk group was decreased by 81.8% at maximum, and triglyceride in the P8BG 1.75 mpk group was decreased by 68.7% at maximum; 5 days after the administration, the decrease of triglyceride in the P8BG 3.5 mpk group, the P8BG 1.75 mpk group and the P8BG 7 mpk group still had statistical difference, while that in the evinacumab 3.5 mpk group and the evinacumab 7 mpk group had no statistical difference; 9 days after the administration, the decrease of triglyceride in the P8BG 7 mpk group and the P8BG 3.5 mpk group still had statistical difference. This indicates that P8BG has more effective and prolonged triglyceride lowering effect than evinacumab at the same dose. In addition, the animals in each group were normal in weight during the experiment.

**Table 7. Effect of P8BG antibody on rat serum triglyceride**

| Antibodies | Evinacumab | | P8BG | | |
|---|---|---|---|---|---|
| Dosage for subcutaneous administration | 7mpk | 3.5mpk | 7mpk | 3.5mpk | 1.75mpk |
| Start TG (mg/dL) | 121.5±17.0 | 122.2±17.1 | 120.1±14.3 | 122.6±15.6 | 120.9±16.1 |
| Minimum TG level | 131.1±12.5 | 109.7±16.7 | 27.6±3.1 | 34.2±4.7 | 72.6±10.9 |
| Max Inh % | 40.7% | 13.1% | 83.7% | 81.8% | 68.7% |

| | | | | | |
|---|---|---|---|---|---|
| Note: Max Inh% refers to the maximum percentage of lipid lowering relative to the TG of the negative control group, and mpk refers to mg/kg. | | | | | |

### 2. In vivo lipid lowering experiment of antibody P3G in rats

SD rats for experiments (aged 4 weeks, SPF, male, purchased from Shanghai Slac Laboratory Animal Co., Ltd.; certification No.: 20170005013017, SCXK (Shanghai) 2017-0005) were subjected to 1 week of adaptive feeding in the laboratory environment (in a 12/12 hour light/dark cycle, at a temperature of 23±1 °C and a humidity of 40-50%), and the animals were provided with standard sterile mouse feed and had free access to food and water. 7 days before the start of the experiment, the animals were fasted for 4 h, and then blood was collected from the orbit and centrifuged at 3500 RPM for 15 min, and serum was collected. Serum lipid concentration (triglycerides) was determined using the ADVIA 2400 chemical system (Siemens). The rats were divided into 4 groups by triglyceride level (hIgG negative control group, evinacumab 3.5 mpk group, P3G 3.5 mpk group and P3G 7 mpk group), 6 rats for each group. The rats in all the groups were each subjected to subcutaneous injection once, and then subjected to blood collection after 4 h of fasting on days 1, 5 and 9, 13 after injection. Serum was separated out, and serum lipid concentration (triglyceride) was determined using the ADVIA 2400 chemical system (Siemens). The experimental data were expressed as Mean±standard deviation (Mean±SEM) and plotted using Graphpad Prism5 software, and TTEST was used for statistical analysis.

The experimental results are shown in FIG. 2. Compared with the hIgG negative control group, 1 day after the administration, triglyceride of each treatment group was decreased, and the degree of decrease of the P3G 3.5mpk group and the P3G 7 mpk group was greater than that of the evinacumab 3.5 mpk group; 9 days after the administration, serum lipid concentration of the P3G 3.5 mpk group and the P3G 7 mpk group remained lower than that of the positive control group.

### Test Example 6: Determination of In Vivo Effect of Anti-ANGPTL3 Antibodies on Plasma Lipid Concentration in High-Fat High-Cholesterol Fed c57bl/6 Mice

c57bl/6 mice (aged 4 weeks, SPF, about 16-18 g, male, purchased from Shanghai Cavens Laboratory Animal Co., Ltd.; certification No.: 201913812, SCXK (Jiangsu) 2016-0010) were subjected to 4 weeks of adaptive feeding (5 mice in each cage) in the laboratory environment (in a 12/12 hour light/dark cycle, at a temperature of 23±1 °C and a humidity of 40-50%), and the animals were fed with normal feed and had free access to food and water. 5 days after pre-blood collection, the common feed was changed to high-fat high-cholesterol feed (D12079B, purchased from Jiangsu Xietong Pharmaceutical Bio-engineering Co., Ltd.) for feeding the mice until the end of the experiment. 8 days before the start of the experiment, the animals were fasted for 4 h , and then blood was collected from the orbit and centrifuged at 8000 RPM for 2 min, and plasma was collected. Plasma lipid concentration was determined using the ADVIA 2400 chemical system (Siemens). The mice were divided into 4 groups by triglyceride level (hIgG negative control group, evinacumab 25 mpk group, P8BG 25 mpk group and P8BG 5 mpk group), 11 mice for each group, and the mice were subjected to subcutaneous injection once a week, 8 times in total. At weeks 2, 3, 5, 7, 9, 10 and 11 after the start of the experiment, the mice in each group were fasted for 4 h, and then blood was collected from the orbit and plasma was collected. Plasma lipid concentration (triglyceride (TG), total cholesterol (TC) and LDL cholesterol (LDL-C)) were determined using the ADVIA 2400 chemical system (Siemens). The experimental data were expressed as Mean±standard deviation (Mean±SEM) and plotted using Graphpad Prism5 software, and TTEST was used for statistical analysis.

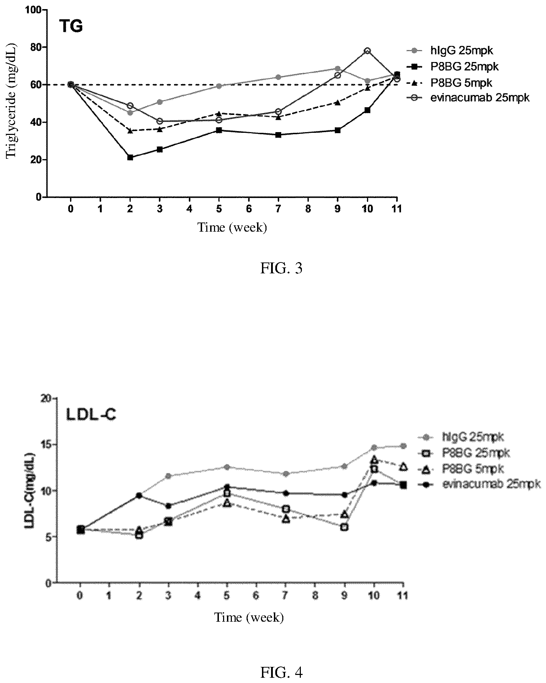

The results are shown in Tables 8-10 and FIGs. 3-5. Compared with the hIgG negative control group, the TG, TC and LDL-C were significantly decreased in the P8BG 25 mpk group 2, 3, 5 and 7 weeks after the start of the experiment; the evinacumab 25 mpk group and the P8BG 5 mpk group showed decrease in TG, TC and LDL-C 3, 5 and 7 weeks after the start of the experiment. 9 weeks after the start of the experiment, only two groups, the P8BG 25 mpk group and the P8BG 5 mpk group, showed significant decrease in triglyceride compared with the hIgG negative control group. Compared with the evinacumab 25 mpk group, the P8BG 25 mpk group showed significantly decreased triglyceride and total cholesterol on weeks 2, 3 and 9 of the experiment; compared with the evinacumab 25 mpk group, the P8BG 25 mpk group showed significantly decreased LDL-C on weeks 2 and 9 of the experiment. Throughout the entire experiment cycle from week 1 to week 11, the TG of the evinacumab 25 mpk group was decreased to as low as 40.5±2.3 at minimum, a 32.60% decrease compared with the start TG; and the TG of the P8BG 25 mpk group was decreased to as low as 21.1±1.3, a 64.90% decrease compared with the start TG. This indicates that the lipid lowering effect of the antibody P8BG is more effective and prolonged than that of evinacumab.

**Table 8. Effect of anti-ANGPTL3 antibody on plasma TG in c57bl/6 mice**

| **Antibodies** | **Evinacumab** | **P8BG** | | **hIgG** |
|---|---|---|---|---|
| Dosage for weekly subcutaneous administration | **25mpk** | **25mpk** | **5mpk** | **25mpk** |
| Start TG (mg/dL) | 60.1±3.2 | 60.1±4.1 | 60.1±3.8 | 60.0±6.2 |
| Minimum TG level | 40.5±2.3 | 21.1±1.3 | 35.5±1.7 | 45.1±7.1 |
| max Inh % | 32.6% | 64.9% | 40.9% | 24.8% |

| | | | | |
|---|---|---|---|---|
| Note: max Inh% refers to the maximum percentage of lipid lowering relative to the start TG | | | | |

**Table 9. Effect of anti-ANGPTL3 antibodies on plasma LDL in c57bl/6 mice**

| **Antibodies** | **Evinacumab** | **P8BG** | |
|---|---|---|---|
| Dosage for weekly subcutaneous administration | **25mpk** | **25mpk** | **5mpk** |
| Max Inh % | 28.10% | 52.10% | 42.80% |

| | | | |
|---|---|---|---|
| Note: Max Inh% refers to the maximum percentage of lipid lowering relative to the LDL of the negative control group | | | |

**Table 10. Effect of anti-ANGPTL3 antibody on plasma TC in c57bl/6 mice**

| **Antibodies** | **Evinacumab** | **P8BG** | |
|---|---|---|---|
| Dosage for weekly subcutaneous administration | **25mpk** | **25mpk** | **5mpk** |
| Max Inh % | 27.50% | 47.90% | 36.20% |

| | | | |
|---|---|---|---|
| Note: Max Inh% refers to the maximum percentage of lipid lowering relative to the TC of the negative control group | | | |

### Test Example 7: Determination of In Vivo Effect of Anti-ANGPTL3 Antibodies on Plasma Lipid Concentration in APOE Mice

APOE mice (aged 8 weeks, SPF, about 22 g, male, purchased from Shanghai Cavens Laboratory Animal Co., Ltd.; certification No.: 201917260, SCXK (Jiangsu) 2016-0010) were subjected to 4 weeks of adaptive feeding (2 mice in each cage) in the laboratory environment (in a 12/12 hour light/dark cycle, at a temperature of 23±1 °C and a humidity of 40-50%), and the animals were fed with high-fat feed (D12108C) and had free access to food and water. 7 days before the start of the experiment, the animals were fasted for 4 h, and then blood was collected from the orbit and centrifuged at 8000 RPM for 2 min, and plasma was collected. Plasma lipid concentration was determined using the ADVIA 2400 chemical system (Siemens). Mice were divided into 5 groups by triglyceride level (hIgG negative control group, evinacumab 10 mpk group, evinacumab 5 mpk group, P8BG 10 mpk group and P8BG 5 mpk group), 8 mice for each group. The mice in all the groups were each subjected to subcutaneous injection once (see Table 11 for administration dosage for each group), and then subjected to blood collection from the orbit after 4 h of fasting on days 1, 7 and 11 after administration. Plasma was collected, and lipid concentrations (triglyceride and HDL cholesterol) were determined using the ADVIA 2400 chemical system (Siemens). The experimental data were expressed as Mean±standard deviation (Mean±SEM) and plotted using Graphpad Prism5 software, and TTEST was used for statistical analysis.

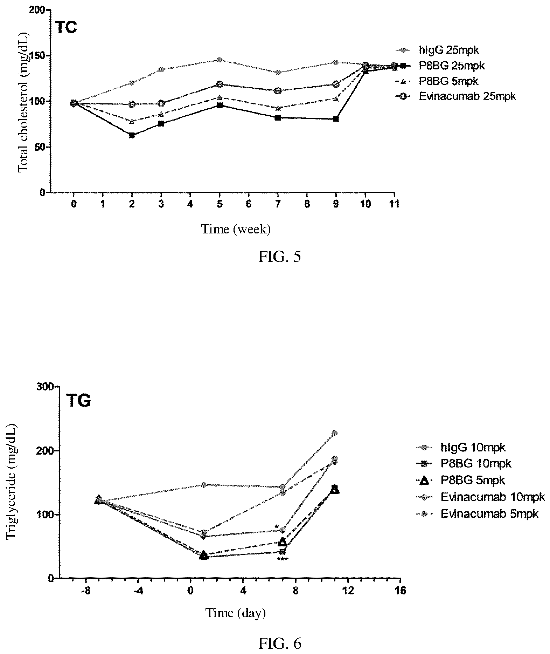

The experimental results are shown in FIGs. 6-7 and Table 11. Compared with the hIgG negative control group, 1 day after the administration, triglyceride in each treatment group was decreased. In the evinacumab (10 mpk) group, the triglyceride was decreased to as low as 65.6±5.4 mg/dL, a 46.2% decrease compared with the start TG value; in the evinacumab (5 mpk) group, the triglyceride was decreased to as low as 71.8±3.3 mg/dL, a 42.1% decrease compared with the start TG value; in the P8BG (10 mpk) group, the triglyceride was decreased to as low as 33.3±2.2 mg/dL, a 72.9% decrease compared with the start TG value; in the P8BG (5 mpk) group, the triglyceride was decreased to as low as 37.0±1.7 mg/dL, a 70.2% decrease compared with the start TG value. In addition, the experimental results show that 1 day after the administration, the plasma HDL-C of P8BG 10 mpk group was increased and the plasma HDL-C of evinacumab 10 mpk group was decreased. However, in the lipid lowering experiment, the decrease in the HDL-C is not generally desired during lipid lowering.

**Table 11. Effect of anti-ANGPTL3 antibodies on plasma triglyceride in APOE mice**

| Antibodies | Evinacumab | | P8BG | | hIgG |
|---|---|---|---|---|---|
| Dosage for subcutaneous administration | 10mpk | 5mpk | 10mpk | 5mpk | 10mpk |
| Start TG (mg/dL) | 122.0±8.8 | 123.9±17.1 | 122.8±10.7 | 124.0±20.8 | 120.5±14.9 |
| Minimum TG level | 65.6±5.4 | 71.8±3.3 | 33.3±2.2 | 37.0±1.7 | 146.7±21.6 |
| max Inh % | 46.20% | 42.10% | 72.90% | 70.20% | -21.70% |

| | | | | | |
|---|---|---|---|---|---|
| Note: max Inh% refers to the maximum percentage of lipid lowering relative to the start TG | | | | | |

### Test Example 8: Evaluation of Pharmacokinetics of Anti-ANGPTL3 Antibodies

### 1. In vivo pharmacokinetic experiment of anti-ANGPTL3 antibodies in mice

C57BL/6 mice (18-24 g, purchased from Beijing Vital River Laboratory Animal Technology Co., Ltd.) were subjected to adaptive feeding (no less than 3 days) in the laboratory environment (in a 12/12 hour light/dark cycle, at a temperature of 16-26 °C and a humidity of 40-70%), and they had free access to food and water during the feeding. The day before the start of the experiment, the C57BL/6 mice were numbered and randomly grouped with 3 mice for each. On the day of experiment, mice in the groups were intravenously injected with test drugs P8BG and evinacumab, respectively, the administration dosage was 10 mg/kg, and the injection volume was 5 mL/kg.

The mice in the administration groups were each subjected to collection of 0.1 mL of whole blood before administration and 5 min, 8 h, 1 d, 2 d, 4 d, 7 d, 10 d, 14 d, 21 d and 28 d after the administration, and the blood was placed at 4 °C for 30 min without anticoagulation and then centrifuged at 1000 g for 15 min. The supernatant (serum) was collected, placed in an EP tube, and stored at -80 °C.

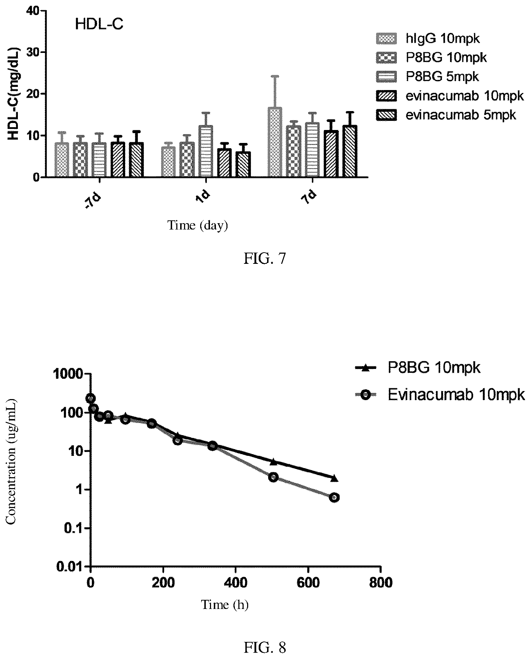

The antibody concentration in serum was determined by ELISA, and the pharmacokinetic parameters were calculated for the test drugs by Winnolin software. The main pharmacokinetic results obtained are shown in Table 12 and FIG. 8. The experimental results show that the P8BG antibody of the present disclosure has a higher half-life in mice than the positive control evinacumab.

**Table 12. Pharmacokinetics of anti-ANGPTL3 antibodies in mice**

| Antibodies | P8BG | Evinacumab |
|---|---|---|
| Route of administration | i.v. | i.v. |
| Administration dosage (mg/kg) | 10 | 10 |
| t1/2h | 131.4±18.8 | 71.1±6.1 |
| t1/2d | 5.47±0.78 | 2.96±0.25 |
| AUC0-∞(h^{∗}ug/ml) | 23272±2887 | 16904±4481 |

| | | |
|---|---|---|
| Note: AUC0-∞: area under the plasma drug concentration-time curve; t1/2 (h): half-life (in hours); t1/2 (d): half-life (in days); i.v.: intravenous injection. | | |

### 2. Evaluation of pharmacokinetics of anti-ANGPTL3 antibody in cynomolgus monkey

Cynomolgus monkeys (3-4 kg, purchased from Guangdong Qianyan Biological Science and Technology Co., Ltd.) were used, and the animals were subjected to at 14 days of quarantine and adaptive feeding before being used in the experiment. They were kept in stainless steel cages with no more than 2 animals per cage. The room temperature of the animal room was controlled to be 18-26 °C, the relative humidity was 40-70%, and the illumination was in a 12/12 light/dark cycle. The animals had free access to water during the experiment. 6 cynomolgus monkeys were randomized into 2 groups with 3 animals per group according to biochemical criteria. The cynomolgus monkeys in the groups were subcutaneously injected with test drugs P8BG and evinacumab, respectively, the administration dosage was 10 mg/kg, and the injection volume was 2 mL/kg.

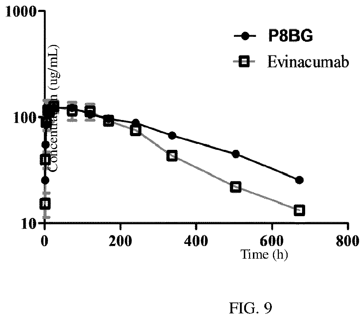

After the grouping, animals were fasted overnight, and they were each subjected to collection of about 2 mL of blood from veins of a lower limb before administration and 1 h, 2 h, 4 h, 8 h, 12 h, 1 d, 3 d, 5 d, 7 d, 10 d, 14 d, 21d, 28 d, 35 d, 42 d, 49 d and 56 d after administration. The blood was placed in a blood collection tube containing separation gel, left to stand at room temperature for 30 min and then centrifuged at 1000 g for 15 min. The supernatant was collected, aliquoted into 2 clean EP tubes and temporarily stored at -70 °C (completed within 2 h after blood collection). The antibody concentration in serum was determined by ELISA, and the pharmacokinetic parameters were calculated for the test drugs by Winnolin software. The main pharmacokinetic results obtained are shown in Table 14 and FIG. 9. The experimental results show that the antibody P8BG of the present disclosure has a higher half-life in cynomolgus monkeys than the positive control evinacumab.

**Table 13. Pharmacokinetics of anti-ANGPTL3 antibodies in cynomolgus monkeys**

| | P8BG | Evinacumab |
|---|---|---|
| Administration dosage (mg/kg) | 10 | 10 |
| Route of administration | sc. | sc. |
| t1/2h | 324.7±115.3 | 157.7±28.6 |
| t1/2d | 13.5±4.8 | 6.6±1.2 |
| AUC 0-∞ (ug/ml^{∗}h) | 44243±7404 | 38027±252 |

| | | |
|---|---|---|
| Note: AUC 0-∞: area under the plasma drug concentration-time curve; t1/2 (h): half-life (in hours); t1/2 (d): half-life (in days); sc.: subcutaneous injection. | | |

## Claims

1. An anti-ANGPTL3 antibody or an antigen-binding fragment thereof, comprising a heavy chain variable region and a light chain variable region of an antibody, wherein:
i) the heavy chain variable region has an HCDR1, an HCDR2 and an HCDR3 identical in sequences to those of a heavy chain variable region set forth in SEQ ID NO: 13, and the light chain variable region has an LCDR1, an LCDR2 and an LCDR3 identical in sequences to those of a light chain variable region set forth in SEQ ID NO: 14;
ii) the heavy chain variable region has an HCDR1, an HCDR2 and an HCDR3 identical in sequences to those of a heavy chain variable region set forth in SEQ ID NO: 11, and the light chain variable region has an LCDR1, an LCDR2 and an LCDR3 identical in sequences to those of a light chain variable region set forth in SEQ ID NO: 12; or
iii) the heavy chain variable region has an HCDR1, an HCDR2 and an HCDR3 identical in sequences to those of a heavy chain variable region set forth in SEQ ID NO: 9, and the light chain variable region has an LCDR1, an LCDR2 and an LCDR3 identical in sequences to those of a light chain variable region set forth in SEQ ID NO: 10.

2. The anti-ANGPTL3 antibody or the antigen-binding fragment thereof according to claim 1, comprising a heavy chain variable region and a light chain variable region, wherein:
iv) the heavy chain variable region comprises an HCDR1, an HCDR2 and an HCDR3 having sequences set forth in SEQ ID NO: 24, SEQ ID NO: 28 and SEQ ID NO: 26, respectively, and the light chain variable region comprises an LCDR1, an LCDR2 and an LCDR3 having sequences set forth in SEQ ID NO: 27, SEQ ID NO: 22 and SEQ ID NO: 23, respectively;
v) the heavy chain variable region comprises an HCDR1, an HCDR2 and an HCDR3 having sequences set forth in SEQ ID NO: 24, SEQ ID NO: 25 and SEQ ID NO: 26, respectively, and the light chain variable region comprises an LCDR1, an LCDR2 and an LCDR3 having sequences set forth in SEQ ID NO: 21, SEQ ID NO: 22 and SEQ ID NO: 23, respectively; or
vi) the heavy chain variable region comprises an HCDR1, an HCDR2 and an HCDR3 having sequences set forth in SEQ ID NO: 18, SEQ ID NO: 19 and SEQ ID NO: 20, respectively, and the light chain variable region comprises an LCDR1, an LCDR2 and an LCDR3 having sequences set forth in SEQ ID NO: 15, SEQ ID NO: 16 and SEQ ID NO: 17, respectively.

3. The anti-ANGPTL3 antibody or the antigen-binding fragment thereof according to claim 1 or 2, comprising a heavy chain variable region and a light chain variable region, wherein:
(A) the heavy chain variable region has at least 90% sequence identity to SEQ ID NO: 13, and/or the light chain variable region has at least 90% sequence identity to SEQ ID NO: 14;
(B) the heavy chain variable region has at least 90% sequence identity to SEQ ID NO: 11, and/or the light chain variable region has at least 90% sequence identity to SEQ ID NO: 12; or
(C) the heavy chain variable region has at least 90% sequence identity to SEQ ID NO: 9, and/or the light chain variable region has at least 90% sequence identity to SEQ ID NO: 10.

4. The anti-ANGPTL3 antibody or the antigen-binding fragment thereof according to claim 3, comprising a heavy chain variable region and a light chain variable region shown below:
(D) a heavy chain variable region having a sequence set forth in SEQ ID NO: 13, and a light chain variable region having a sequence set forth in SEQ ID NO: 14;
(E) a heavy chain variable region having a sequence set forth in SEQ ID NO: 11, and a light chain variable region having a sequence set forth in SEQ ID NO: 12; or
(F) a heavy chain variable region having a sequence set forth in SEQ ID NO: 9, and a light chain variable region having a sequence set forth in SEQ ID NO: 10.

5. The anti-ANGPTL3 antibody or the antigen-binding fragment thereof according to any one of claims 1 to 4, wherein the anti-ANGPTL3 antibody further comprises a heavy chain constant region and a light chain constant region;
preferably, the heavy chain constant region is selected from the group consisting of human IgG1, IgG2, IgG3 and IgG4 constant regions, and the light chain constant region is selected from the group consisting of human κ chain and λ chain constant regions;
more preferably, the heavy chain constant region has a sequence set forth in SEQ ID NO: 29 or a sequence having at least 85% sequence identity to SEQ ID NO: 29, and/or the light chain constant region has a sequence set forth in SEQ ID NO: 30 or a sequence having at least 85% sequence identity to SEQ ID NO: 30.

6. The anti-ANGPTL3 antibody or the antigen-binding fragment thereof according to any one of claims 1 to 5, wherein the anti-ANGPTL3 antibody comprises a heavy chain and a light chain, wherein,
(G) the heavy chain has at least 85% sequence identity to SEQ ID NO: 35, and/or the light chain has at least 85% sequence identity to SEQ ID NO: 36;
(H) the heavy chain has at least 85% sequence identity to SEQ ID NO: 33, and/or the light chain has at least 85% sequence identity to SEQ ID NO: 34; or
(I) the heavy chain has at least 85% sequence identity to SEQ ID NO: 31, and/or the light chain has at least 85% sequence identity to SEQ ID NO: 32.

7. The anti-ANGPTL3 antibody or the antigen-binding fragment thereof according to any one of claims 1 to 6, wherein the anti-ANGPTL3 antibody comprises a heavy chain and a light chain, wherein,
(J) the heavy chain is set forth in SEQ ID NO: 35, and the light chain is set forth in SEQ ID NO: 36;
(K) the heavy chain is set forth in SEQ ID NO: 33, and the light chain is set forth in SEQ ID NO: 34; or
(L) the heavy chain is set forth in SEQ ID NO: 31, and the light chain is set forth in SEQ ID NO: 32.

8. An isolated anti-ANGPTL3 antibody or an antigen-binding fragment thereof, wherein the antibody competes with the anti-ANGPTL3 antibody or the antigen-binding fragment thereof according to any one of claims 1 to 7 for binding to an ANGPTL3 antigen.

9. A nucleic acid molecule encoding the anti-ANGPTL3 antibody or the antigen-binding fragment thereof according to any one of claims 1 to 8.

10. A host cell comprising the nucleic acid molecule according to claim 9.

11. A pharmaceutical composition, comprising:
(A) a therapeutically effective amount of the anti-ANGPTL3 antibody or the antigen-binding fragment thereof according to any one of claims 1 to 8, or the nucleic acid molecule according to claim 9, and
(B) one or more pharmaceutically acceptable carriers, diluents, buffers or excipients.

12. A method for immunodetection or determination of ANGPTL3, comprising the step of using the anti-ANGPTL3 antibody or the antigen-binding fragment thereof according to any one of claims 1 to 8.

13. A kit, comprising the anti-ANGPTL3 antibody or the antigen-binding fragment thereof according to any one of claims 1 to 8.

14. A method for treating a disease or a disorder, comprising administering to a subject a therapeutically effective amount of the anti-ANGPTL3 antibody or the antigen-binding fragment thereof according to any one of claims 1 to 8, the nucleic acid molecule according to claim 9, or the pharmaceutical composition according to claim 11, wherein preferably, the disease or the disorder is an ANGPTL3-associated disease or disorder; more preferably, the disease or the disorder is hypercholesterolemia, hyperlipidemia or an atherosclerotic disease.
